# EUROPEAN PATENT APPLICATION

(11) **EP 3 581 200 A1**
(43) Date of publication of application: **18.12.2019**
(21) Application number: 18177502.4
(22) Date of filing: 13.06.2018
(51) Int. Cl.: A61K 39/00, C07K 14/725, C07K 14/705, C07K 16/28

(54) **REVERSED UNIVERSAL CHIMERIC ANTIGEN RECEPTOR EXPRESSING IMMUNE CELLS FOR TARGETING OF DIVERSE MULTIPLE ANTIGENS AND METHOD OF MANUFACTURING THE SAME AND USE OF THE SAME FOR TREATMENT OF CANCER, INFECTIONS AND AUTOIMMUNE DISORDERS**

(71) Applicant: GEMoaB Monoclonals GmbH, 01307 Dresden (DE); Technische Universität Dresden, 01069 Dresden (DE)
(72) Inventor: Bachmann, Michael, 65779 Kelkheim (DE)
(74) Representative: Kailuweit & Uhlemann Patentanwälte Partnerschaft mbB

(57) **Abstract**

The present invention relates to immune cell-based anti-cancer therapeutics and methods of using the therapeutics in the treatment of cancer.

SEQUENCE LISTING

<110>  GEMoaB Monoclonals GmbH

<120>  Reversed universal chimeric antigen receptor expressing immune
       cells for targeting of diverse multiple antigens and method of
       manufacturing the same and use of the same for treatment of
       cancer, infections and autoimmune disorders

<130>  01281P0017EP

<160>  39

<170>  PatentIn version 3.5

<210>  1
<211>  1101
<212>  DNA
<213>  Artificial Sequence

<220>
<223>  Homo sapiens

<400>  1
atgcgccgca tgcagctgct gcttctgatc gctctgagcc tggctcttgt gaccaactct      60

gaattcaaac ccctacctga agtgactgat gagtatgctg ccgctgggcc cggaggaggc     120

ggcagcaaga tcctggtcaa acagtcccct atgctggtcg cttacgacaa cgccgttaat     180

ctgagttgca aatatagtta caacctgttt agccgggaat ttcgcgcatc tctccacaag     240

ggactggatt ctgcggttga ggtttgtgtg gtctatggca attatagcca gcaactgcaa     300

gtgtacagca aaacaggctt taactgcgac gggaaactcg ggaacgaatc agtgaccttc     360

tatctgcaga acctgtacgt taaccaaaca gatatttact tctgcaagat agaggtgatg     420

gctccaccgc cagcactgga taacgagaag tccaatggaa ccatcattca cgtcaagggg     480

aagcatctgt gtccttcccc gttgttccct gggccgagca aacccttttg ggtgcttgtg     540

gtagttggcg gggtattggc ctgctattcc cttctcgtaa ctgtggcctt catcatcttc     600

tgggtcagat ctaagaggtc taggggcggg catagcgact acatgaacat gacacccagg     660

cggcctggcc ccactcgcaa acactaccag ccatacgcac caccaagaga ctttgccgca     720

tatcggagtg gtggcggcgg gtcaggaggt ggagctagcg gtggaggagg ttccttctct     780

aggtcagctg atgctcccgc ctatcagcaa ggtcagaacc agctctacaa tgagctgaat     840

ctgggacgtc gggaggagta cgacgtgctg gataaacgaa gaggacgcga tcccgagatg     900

ggtgggaagc ctaggcgcaa gaatccccag gaaggcctct acaatgaact gcagaaagac     960

aagatggccg aagcctacag cgagattggc atgaaagggg agcgacggag aggaaaggga    1020

catgacgggt tgtatcaggg tctttccact gcgacaaagg atacctatgg ggctctgcac    1080

atgcaagcac tgccacctag a                                              1101

<210>  2
<211>  60
<212>  DNA
<213>  Homo sapiens

<400>  2
atgcgccgca tgcagctgct gcttctgatc gctctgagcc tggctcttgt gaccaactct        60


<210>  3
<211>  30
<212>  DNA
<213>  Homo sapiens

<400>  3
aaacccctac ctgaagtgac tgatgagtat                                          30


<210>  4
<211>  399
<212>  DNA
<213>  Homo sapiens

<400>  4
aagatcctgg tcaaacagtc ccctatgctg gtcgcttacg acaacgccgt taatctgagt        60

tgcaaatata gttacaacct gtttagccgg gaatttcgcg catctctcca caagggactg       120

gattctgcgg ttgaggtttg tgtggtctat ggcaattata gccagcaact gcaagtgtac       180

agcaaaacag gctttaactg cgacgggaaa ctcgggaacg aatcagtgac cttctatctg       240

cagaacctgt acgttaacca aacagatatt tacttctgca agatagaggt gatggctcca       300

ccgccagcac tggataacga gaagtccaat ggaaccatca ttcacgtcaa ggggaagcat       360

ctgtgtcctt ccccgttgtt ccctgggccg agcaaaccc                              399


<210>  5
<211>  81
<212>  DNA
<213>  Homo sapiens

<400>  5
ttttgggtgc ttgtggtagt tggcggggta ttggcctgct attcccttct cgtaactgtg        60

gccttcatca tcttctgggt c                                                   81


<210>  6
<211>  123
<212>  DNA
<213>  Homo sapiens

<400>  6
agatctaaga ggtctagggg cgggcatagc gactacatga acatgacacc caggcggcct        60

ggccccactc gcaaacacta ccagccatac gcaccaccaa gagactttgc cgcatatcgg       120

agt                                                                      123

<210>  7
<211>  327
<212>  DNA
<213>  Homo sapiens

<400>  7
ttctctaggt cagctgatgc tcccgcctat cagcaaggtc agaaccagct ctacaatgag      60

ctgaatctgg gacgtcggga ggagtacgac gtgctggata aacgaagagg acgcgatccc     120

gagatgggtg ggaagcctag gcgcaagaat ccccaggaag gcctctacaa tgaactgcag     180

aaagacaaga tggccgaagc ctacagcgag attggcatga aaggggagcg acggagagga     240

aagggacatg acgggttgta tcagggtctt tccactgcga caaaggatac ctatggggct     300

ctgcacatgc aagcactgcc acctaga                                         327


<210>  8
<211>  1137
<212>  DNA
<213>  Artificial Sequence

<220>
<223>  Homo sapiens

<400>  8
atgcgccgca tgcagctgct gcttctgatc gctctgagcc tggctcttgt gaccaactct      60

gaattcaaac cctacctgaa agtgactgat gagtatgctg ccgctgggcc cggaggaggc     120

ggcagcaaga tcctggtcaa acagtcccct atgctggtcg cttacgacaa cgccgttaat     180

ctgagttgca aatatagtta caacctgttt agccgggaat ttcgcgcatc tctccacaag     240

ggactggatt ctgcggttga ggtttgtgtg gtctatggca attatagcca gcaactgcaa     300

gtgtacagca aaacaggctt taactgcgac gggaaactcg ggaacgaatc agtgaccttc     360

tatctgcaga acctgtacgt taaccaaaca gatatttact tctgcaagat agaggtgatg     420

gctccaccgc cagcactgga taacgagaag tccaatggaa ccatcattca cgtcaagggg     480

aagcatctgt gtccttcccc gttgttccct gggccgagca aaccctttgg ggtgcttgtg     540

gtagttggcg gggtattggc ctgctattcc cttctcgtaa ctgtggcctt catcatcttc     600

tgggtcagat ctaagaggtc taggggcggg ggcgggtcaa aacgcggacg gaagaaactg     660

ctgtacatct tcaagcagcc cttcatgcgc cccgtgcaga caacacagga agaggacggt     720

tgcagctgcc gatttcccga agaggaggag ggaggctgtg aattgggtgg cggcgggtca     780

ggaggtggag ctagcggtgg aggaggttcc ttctctaggt cagctgatgc tcccgcctat     840

cagcaaggtc agaaccagct ctacaatgag ctgaatctgg gacgtcggga ggagtacgac     900

gtgctggata aacgaagagg acgcgatccc gagatgggtg ggaagcctag gcgcaagaat     960

ccccaggaag gcctctacaa tgaactgcag aaagacaaga tggccgaagc ctacagcgag    1020

attggcatga aaggggagcg acggagagga aagggacatg acgggttgta tcagggtctt    1080

tccactgcga caaaggatac ctatggggct ctgcacatgc aagcactgcc acctaga 1137

<210> 9
<211> 126
<212> DNA
<213> Homo sapiens

<400> 9
aaacgcggac ggaagaaact gctgtacatc ttcaagcagc ccttcatgcg ccccgtgcag 60

acaacacagg aagaggacgg ttgcagctgc cgatttcccg aagaggagga gggaggctgt 120

gaattg 126

<210> 10
<211> 1125
<212> DNA
<213> Artificial Sequence

<220>
<223> Homo sapiens

<400> 10
atgcgccgca tgcagctgct gcttctgatc gctctgagcc tggctcttgt gaccaactct 60

gaattcgaga aagaagcact gaagaaaata atagaagacc aacaagaatc cctaaacaaa 120

gctgccgctg ggcccggagg aggcggcagc aagatcctgg tcaaacagtc ccctatgctg 180

gtcgcttacg acaacgccgt taatctgagt tgcaaatata gttacaacct gtttagccgg 240

gaatttcgcg catctctcca caagggactg gattctgcgg ttgaggtttg tgtggtctat 300

ggcaattata gccagcaact gcaagtgtac agcaaaacag gctttaactg cgacgggaaa 360

ctcgggaacg aatcagtgac cttctatctg cagaacctgt acgttaacca aacagatatt 420

tacttctgca agatagaggt gatggctcca ccgccagcac tggataacga gaagtccaat 480

ggaaccatca ttcacgtcaa ggggaagcat ctgtgtcctt ccccgttgtt ccctggcccg 540

agcaaaccct tttgggtgct tgtggtagtt ggcggggtat tggcctgcta ttcccttctc 600

gtaactgtgg ccttcatcat cttctgggtc agatctaaga ggtctagggg cgggcatagc 660

gactacatga acatgacacc caggcggcct ggccccactc gcaaacacta ccagccatac 720

gcaccaccaa gagactttgc cgcatatcgg agtggtggcg gcgggtcagg aggtggagct 780

agcggtggag gaggttcctt ctctaggtca gctgatgctc ccgcctatca gcaaggtcag 840

aaccagctct acaatgagct gaatctggga cgtcgggagg agtacgacgt gctggataaa 900

cgaagaggac gcgatcccga gatgggtggg aagcctaggc gcaagaatcc ccaggaaggc 960

ctctacaatg aactgcagaa agacaagatg gccgaagcct acagcgagat tggcatgaaa 1020

ggggagcgac ggagaggaaa gggacatgac gggttgtatc agggtctttc cactgcgaca 1080

aaggatacct atggggctct gcacatgcaa gcactgccac ctaga 1125

<210>  11
<211>  54
<212>  DNA
<213>  Homo sapiens

<400>  11
gagaaagaag cactgaagaa aataatagaa gaccaacaag aatccctaaa caaa          54


<210>  12
<211>  1161
<212>  DNA
<213>  Artificial Sequence

<220>
<223>  Homo sapiens

<400>  12
atgcgccgca tgcagctgct gcttctgatc gctctgagcc tggctcttgt gaccaactct      60

gaattcgaga aagaagcact gaagaaaata atagaagacc aacaagaatc cctaaacaaa     120

gctgccgctg ggcccggagg aggcggcagc aagatcctgg tcaaacagtc ccctatgctg     180

gtcgcttacg acaacgccgt taatctgagt tgcaaatata gttacaacct gtttagccgg     240

gaatttcgcg catctctcca caagggactg gattctgcgg ttgaggtttg tgtggtctat     300

ggcaattata gccagcaact gcaagtgtac agcaaaacag gctttaactg cgacgggaaa     360

ctcgggaacg aatcagtgac cttctatctg cagaacctgt acgttaacca aacagatatt     420

tacttctgca agatagaggt gatggctcca ccgccagcac tggataacga gaagtccaat     480

ggaaccatca ttcacgtcaa ggggaagcat ctgtgtcctt ccccgttgtt ccctgggccg     540

agcaaaccct tttgggtgct tgtggtagtt ggcggggtat tggcctgcta ttcccttctc     600

gtaactgtgg ccttcatcat cttctgggtc agatctaaga ggtctagggg cgggggcggg     660

tcaaaacgcg gacggaagaa actgctgtac atcttcaagc agcccttcat gcgccccgtg     720

cagacaacac aggaagagga cggttgcagc tgccgatttc ccgaagagga ggagggaggc     780

tgtgaattgg gtggcggcgg gtcaggaggt ggagctagcg gtggaggagg ttccttctct     840

aggtcagctg atgctcccgc ctatcagcaa ggtcagaacc agctctacaa tgagctgaat     900

ctgggacgtc gggaggagta cgacgtgctg gataaacgaa gaggacgcga tcccgagatg     960

ggtgggaagc ctaggcgcaa gaatccccag gaaggcctct acaatgaact gcagaaagac    1020

aagatggccg aagcctacag cgagattggc atgaaagggg agcgacggag aggaaaggga    1080

catgacgggt tgtatcaggg tctttccact gcgacaaagg atacctatgg ggctctgcac    1140

atgcaagcac tgccacctag a                                              1161


<210>  13
<211>  63

<212>  DNA
<213>  Homo sapiens

<400>  13
atggagacag acacactcct gctatgggta ctgctgctct gggttccagg ttccactggt      60

gac                                                                    63


<210>  14
<211>  360
<212>  DNA
<213>  Artificial Sequence

<220>
<223>  Mouse human chimera

<400>  14
caggtgcagc tggtgcagag cggagccgag gtgaagaagc ctggagcctc tgtgaaggtg      60

agctgcaagg cttctggcta caccttcacc cactactaca tctactgggt gagacaggct     120

cccggacagg gcctggagtg gatgggaggc gtgaacccca gcaacggagg cacccacttc     180

aacgagaagt tcaagtctcg cgtgaccatg acccgcgaca ccagcatctc taccgcttac     240

atggagctga gccgcctgcg ctctgatgat accgctgtgt actactgcgc tcgcagcgag     300

tacgattacg gactgggctt cgcctactgg ggccagggaa ccctggtgac cgtgagctct     360


<210>  15
<211>  336
<212>  DNA
<213>  Artificial Sequence

<220>
<223>  Mouse human chimera

<400>  15
gatatcgtga tgacccagtc tcctgatagc ctggctgtga gcctgggcga gagagctacc      60

atcaactgca agagcagcca gagcctgctg aactctcgca cccctaagaa ctaccttgct     120

tggtaccagc agaagcctgg acagccccct aagctgctga tctactgggc ttctacccgc     180

aagagcggcg tgcccgacag attctctggc agcggaagcg gcaccgattt caccctgacc     240

atcagcagcc tgcaggctga ggacgtggcc gtgtactact gcaagcagtc ttacaacctg     300

ctgaccttcg gaggcggaac caaggtggag atcaag                               336


<210>  16
<211>  30
<212>  DNA
<213>  Homo sapiens

<400>  16
gaacaaaaac tcatctcaga agaggatctg                                       30


<210>  17

<211>  18
<212>  DNA
<213>  Artificial Sequence

<220>
<223>  Hexahistidine

<400>  17
catcatcatc atcatcat                                                    18


<210>  18
<211>  336
<212>  DNA
<213>  Artificial Sequence

<220>
<223>  Mouse human chimera

<400>  18
gacattgtta tgacccagag cccggactct ctcgctgtta gtcttggtga gcgagcgact      60

attaactgcc ggagcagtca gagtttgttg gactctcgga cgaaaaagaa ctacctggca     120

tggtaccagc agaagccggg ccaaccacct aaattactga tatattgggc gtcgactcgt     180

gagtcagggg taccggacag gttttctgga agcggatcag gaacagactt cactttgacg     240

atctcttcgc ttcaagccga ggacgttgcg gtttattatt gtaagcaaag ctataatctg     300

ccgacatttg gtggcggcac caaggttgaa attaag                               336


<210>  19
<211>  354
<212>  DNA
<213>  Artificial Sequence

<220>
<223>  Mouse human chimera

<400>  19
gaggtgcaac tggtcgaaag tggcggtggt ttagttcagc ctggtggaag tctacggctt      60

agctgcgcag catccggttt cacctttagc gacttttgga tgaactgggt tcggcaggct     120

ccgggcaaag gactggagtg ggttgggcaa atccgcaaca aaccgaataa ctacgaaact     180

tattactcag atagcctgaa gggtcgattc accatcagca gggatgattc aaagtcaatc     240

acttacctac agatgaactc attaagagcg gaggatactg cggtgtatta ctgtacacta     300

ggtaactcct ggttcgcgta ttggggacag ggcacccttg taaccgtctc cagc           354


<210>  20
<211>  345
<212>  DNA
<213>  Artificial Sequence

<220>
<223>  Human mouse chimera

<400>  20
gaggtgcagc tgcagcagtc aggacctgaa ctggtgaagc ctgggacttc agtgaggata      60

tcctgcaaga cttctggata cacattcact gaatatacca tacactgggt gaagcagagc     120

catggaaaga gccttgagtg gattggaaac atcaatccta acaatggtgg taccacctac     180

aatcagaagt tcgaggacaa ggccacattg actgtagaca agtcctccag tacagcctac     240

atggagctcc gcagcctaac atctgaggat tctgcagtct attattgtgc agctggttgg     300

aactttgact actggggcca agggaccacg gtcaccgtct cctca                     345


<210>  21
<211>  321
<212>  DNA
<213>  Artificial Sequence

<220>
<223>  Human mouse chimera

<400>  21
gacattgtga tgacccagtc tcacaaattc atgtccacat cagtaggaga cagggtcagc      60

atcatctgta aggccagtca agatgtgggt actgctgtag actggtatca acagaaacca     120

ggacaatctc ctaaactact gatttattgg gcatccactc ggcacactgg agtccctgat     180

cgcttcacag gcagtggatc tgggacagac ttcactctca ccattactaa tgttcagtct     240

gaagacttgg cagattattt ctgtcagcaa tataacagct atcccctcac gttcggtgct     300

gggaccatgc tggacctgaa a                                               321


<210>  22
<211>  357
<212>  DNA
<213>  Artificial Sequence

<220>
<223>  Human mouse chimera

<400>  22
gaagtgcagc tgcagcagtc tggccccgag ctggtcaaac caggcgccag cgtgaagatg      60

agctgcaagg ccagcggcta caccttcacc gactactaca tgaagtgggt caagcagagc     120

cacggcaaga gcctggaatg gatcggcgac atcatcccca gcaacggcgc caccttctac     180

aaccagaagt tcaagggcaa ggccaccctg accgtggaca gaagcagcag caccgcctac     240

atgcacctga acagcctgac cagcgaggac agcgccgtgt actactgcac cagaagccat     300

ctgctgcggg ccagttggtt cgcttattgg ggccagggca ccctggtcac agtgtct        357


<210>  23
<211>  339
<212>  DNA
<213>  Artificial Sequence

<220>
<223>  Human mouse chimera

<400>  23
gacttcgtga tgacccagag ccctagcagc ctgaccgtga cagccggcga gaaagtgacc      60

atgagctgca agagcagcca gagcctgctg aactccggca accagaagaa ctacctgacc     120

tggtatctgc agaagcccgg acagcccccc aagctgctga tctactgggc cagcaccaga     180

gaaagcggcg tgcccgatag attcacaggc agcggcagcg gcaccgactt caccctgaca     240

atcagcagcg tgcaggccga ggacctggcc gtgtactatt gccagaacga ctacagctac     300

ccctacacct tcggaggcgg gaccaagctg gaaatcaag                            339


<210>  24
<211>  367
<212>  PRT
<213>  Artificial Sequence

<220>
<223>  Homo sapiens

<400>  24

Met Arg Arg Met Gln Leu Leu Leu Leu Ile Ala Leu Ser Leu Ala Leu
1               5                   10                  15


Val Thr Asn Ser Glu Phe Lys Pro Leu Pro Glu Val Thr Asp Glu Tyr
            20                  25                  30


Ala Ala Ala Gly Pro Gly Gly Gly Gly Ser Lys Ile Leu Val Lys Gln
        35                  40                  45


Ser Pro Met Leu Val Ala Tyr Asp Asn Ala Val Asn Leu Ser Cys Lys
    50                  55                  60


Tyr Ser Tyr Asn Leu Phe Ser Arg Glu Phe Arg Ala Ser Leu His Lys
65                  70                  75                  80


Gly Leu Asp Ser Ala Val Glu Val Cys Val Val Tyr Gly Asn Tyr Ser
                85                  90                  95


Gln Gln Leu Gln Val Tyr Ser Lys Thr Gly Phe Asn Cys Asp Gly Lys
            100                 105                 110


Leu Gly Asn Glu Ser Val Thr Phe Tyr Leu Gln Asn Leu Tyr Val Asn
        115                 120                 125


Gln Thr Asp Ile Tyr Phe Cys Lys Ile Glu Val Met Ala Pro Pro Pro
    130                 135                 140

Ala Leu Asp Asn Glu Lys Ser Asn Gly Thr Ile Ile His Val Lys Gly
145                 150                 155                 160

Lys His Leu Cys Pro Ser Pro Leu Phe Pro Gly Pro Ser Lys Pro Phe
                165                 170                 175

Trp Val Leu Val Val Val Gly Gly Val Leu Ala Cys Tyr Ser Leu Leu
            180                 185                 190

Val Thr Val Ala Phe Ile Ile Phe Trp Val Arg Ser Lys Arg Ser Arg
        195                 200                 205

Gly Gly His Ser Asp Tyr Met Asn Met Thr Pro Arg Arg Pro Gly Pro
    210                 215                 220

Thr Arg Lys His Tyr Gln Pro Tyr Ala Pro Pro Arg Asp Phe Ala Ala
225                 230                 235                 240

Tyr Arg Ser Gly Gly Gly Gly Ser Gly Gly Gly Ala Ser Gly Gly Gly
                245                 250                 255

Gly Ser Phe Ser Arg Ser Ala Asp Ala Pro Ala Tyr Gln Gln Gly Gln
            260                 265                 270

Asn Gln Leu Tyr Asn Glu Leu Asn Leu Gly Arg Arg Glu Glu Tyr Asp
        275                 280                 285

Val Leu Asp Lys Arg Arg Gly Arg Asp Pro Glu Met Gly Gly Lys Pro
    290                 295                 300

Arg Arg Lys Asn Pro Gln Glu Gly Leu Tyr Asn Glu Leu Gln Lys Asp
305                 310                 315                 320

Lys Met Ala Glu Ala Tyr Ser Glu Ile Gly Met Lys Gly Glu Arg Arg
                325                 330                 335

Arg Gly Lys Gly His Asp Gly Leu Tyr Gln Gly Leu Ser Thr Ala Thr
            340                 345                 350

Lys Asp Thr Tyr Gly Ala Leu His Met Gln Ala Leu Pro Pro Arg
        355                 360                 365

<210>  25
<211>  379
<212>  PRT
<213>  Artificial Sequence

<220>
<223>  Homo sapiens

<400>  25

Met Arg Arg Met Gln Leu Leu Leu Leu Ile Ala Leu Ser Leu Ala Leu
1               5                   10                  15

Val Thr Asn Ser Glu Phe Lys Pro Leu Pro Glu Val Thr Asp Glu Tyr
            20                  25                  30

Ala Ala Ala Gly Pro Gly Gly Gly Gly Ser Lys Ile Leu Val Lys Gln
        35                  40                  45

Ser Pro Met Leu Val Ala Tyr Asp Asn Ala Val Asn Leu Ser Cys Lys
    50                  55                  60

Tyr Ser Tyr Asn Leu Phe Ser Arg Glu Phe Arg Ala Ser Leu His Lys
65                  70                  75                  80

Gly Leu Asp Ser Ala Val Glu Val Cys Val Val Tyr Gly Asn Tyr Ser
                85                  90                  95

Gln Gln Leu Gln Val Tyr Ser Lys Thr Gly Phe Asn Cys Asp Gly Lys
            100                 105                 110

Leu Gly Asn Glu Ser Val Thr Phe Tyr Leu Gln Asn Leu Tyr Val Asn
        115                 120                 125

Gln Thr Asp Ile Tyr Phe Cys Lys Ile Glu Val Met Ala Pro Pro Pro
    130                 135                 140

Ala Leu Asp Asn Glu Lys Ser Asn Gly Thr Ile Ile His Val Lys Gly
145                 150                 155                 160

Lys His Leu Cys Pro Ser Pro Leu Phe Pro Gly Pro Ser Lys Pro Phe
                165                 170                 175

Trp Val Leu Val Val Val Gly Gly Val Leu Ala Cys Tyr Ser Leu Leu
            180                 185                 190

Val Thr Val Ala Phe Ile Ile Phe Trp Val Arg Ser Lys Arg Ser Arg
        195                 200                 205

Gly Gly Gly Gly Ser Lys Arg Gly Arg Lys Lys Leu Leu Tyr Ile Phe
    210                 215                 220

Lys Gln Pro Phe Met Arg Pro Val Gln Thr Thr Gln Glu Glu Asp Gly
225                 230                 235                 240

Cys Ser Cys Arg Phe Pro Glu Glu Glu Glu Gly Gly Cys Glu Leu Gly
                245                 250                 255

Gly Gly Gly Ser Gly Gly Gly Ala Ser Gly Gly Gly Gly Ser Phe Ser
            260                 265                 270

Arg Ser Ala Asp Ala Pro Ala Tyr Gln Gln Gly Gln Asn Gln Leu Tyr
        275                 280                 285

Asn Glu Leu Asn Leu Gly Arg Arg Glu Glu Tyr Asp Val Leu Asp Lys
    290                 295                 300

Arg Arg Gly Arg Asp Pro Glu Met Gly Gly Lys Pro Arg Arg Lys Asn
305                 310                 315                 320

Pro Gln Glu Gly Leu Tyr Asn Glu Leu Gln Lys Asp Lys Met Ala Glu
                325                 330                 335

Ala Tyr Ser Glu Ile Gly Met Lys Gly Glu Arg Arg Arg Gly Lys Gly
            340                 345                 350

His Asp Gly Leu Tyr Gln Gly Leu Ser Thr Ala Thr Lys Asp Thr Tyr
        355                 360                 365

Gly Ala Leu His Met Gln Ala Leu Pro Pro Arg
    370                 375

<210>  26
<211>  375
<212>  PRT
<213>  Artificial Sequence

<220>
<223>  Homo sapiens

<400>  26

Met Arg Arg Met Gln Leu Leu Leu Leu Ile Ala Leu Ser Leu Ala Leu
1               5                   10                  15

Val Thr Asn Ser Glu Phe Glu Lys Glu Ala Leu Lys Lys Ile Ile Glu
            20                  25                  30

Asp Gln Gln Glu Ser Leu Asn Lys Ala Ala Ala Gly Pro Gly Gly Gly
        35                  40                  45

Gly Ser Lys Ile Leu Val Lys Gln Ser Pro Met Leu Val Ala Tyr Asp
    50                  55                  60

Asn Ala Val Asn Leu Ser Cys Lys Tyr Ser Tyr Asn Leu Phe Ser Arg

65                  70                  75                  80

Glu Phe Arg Ala Ser Leu His Lys Gly Leu Asp Ser Ala Val Glu Val
                85                  90                  95

Cys Val Val Tyr Gly Asn Tyr Ser Gln Gln Leu Gln Val Tyr Ser Lys
            100                 105                 110

Thr Gly Phe Asn Cys Asp Gly Lys Leu Gly Asn Glu Ser Val Thr Phe
        115                 120                 125

Tyr Leu Gln Asn Leu Tyr Val Asn Gln Thr Asp Ile Tyr Phe Cys Lys
    130                 135                 140

Ile Glu Val Met Ala Pro Pro Pro Ala Leu Asp Asn Glu Lys Ser Asn
145                 150                 155                 160

Gly Thr Ile Ile His Val Lys Gly Lys His Leu Cys Pro Ser Pro Leu
                165                 170                 175

Phe Pro Gly Pro Ser Lys Pro Phe Trp Val Leu Val Val Val Gly Gly
            180                 185                 190

Val Leu Ala Cys Tyr Ser Leu Leu Val Thr Val Ala Phe Ile Ile Phe
        195                 200                 205

Trp Val Arg Ser Lys Arg Ser Arg Gly Gly His Ser Asp Tyr Met Asn
    210                 215                 220

Met Thr Pro Arg Arg Pro Gly Pro Thr Arg Lys His Tyr Gln Pro Tyr
225                 230                 235                 240

Ala Pro Pro Arg Asp Phe Ala Ala Tyr Arg Ser Gly Gly Gly Gly Ser
                245                 250                 255

Gly Gly Gly Ala Ser Gly Gly Gly Gly Ser Phe Ser Arg Ser Ala Asp
            260                 265                 270

Ala Pro Ala Tyr Gln Gln Gly Gln Asn Gln Leu Tyr Asn Glu Leu Asn
        275                 280                 285

Leu Gly Arg Arg Glu Glu Tyr Asp Val Leu Asp Lys Arg Arg Gly Arg
    290                 295                 300

Asp Pro Glu Met Gly Gly Lys Pro Arg Arg Lys Asn Pro Gln Glu Gly
305                 310                 315                 320

Leu Tyr Asn Glu Leu Gln Lys Asp Lys Met Ala Glu Ala Tyr Ser Glu
                325                 330                 335

Ile Gly Met Lys Gly Glu Arg Arg Arg Gly Lys Gly His Asp Gly Leu
            340                 345                 350

Tyr Gln Gly Leu Ser Thr Ala Thr Lys Asp Thr Tyr Gly Ala Leu His
        355                 360                 365

Met Gln Ala Leu Pro Pro Arg
    370                 375

<210>  27
<211>  387
<212>  PRT
<213>  Artificial Sequence

<220>
<223>  Homo sapiens

<400>  27

Met Arg Arg Met Gln Leu Leu Leu Leu Ile Ala Leu Ser Leu Ala Leu
1               5                   10                  15

Val Thr Asn Ser Glu Phe Glu Lys Glu Ala Leu Lys Lys Ile Ile Glu
            20                  25                  30

Asp Gln Gln Glu Ser Leu Asn Lys Ala Ala Ala Gly Pro Gly Gly Gly
        35                  40                  45

Gly Ser Lys Ile Leu Val Lys Gln Ser Pro Met Leu Val Ala Tyr Asp
    50                  55                  60

Asn Ala Val Asn Leu Ser Cys Lys Tyr Ser Tyr Asn Leu Phe Ser Arg
65                  70                  75                  80

Glu Phe Arg Ala Ser Leu His Lys Gly Leu Asp Ser Ala Val Glu Val
                85                  90                  95

Cys Val Val Tyr Gly Asn Tyr Ser Gln Gln Leu Gln Val Tyr Ser Lys
            100                 105                 110

Thr Gly Phe Asn Cys Asp Gly Lys Leu Gly Asn Glu Ser Val Thr Phe
        115                 120                 125

Tyr Leu Gln Asn Leu Tyr Val Asn Gln Thr Asp Ile Tyr Phe Cys Lys
    130                 135                 140

Ile Glu Val Met Ala Pro Pro Pro Ala Leu Asp Asn Glu Lys Ser Asn

145                 150                 155                 160

Gly Thr Ile Ile His Val Lys Gly Lys His Leu Cys Pro Ser Pro Leu
                165                 170                 175

Phe Pro Gly Pro Ser Lys Pro Phe Trp Val Leu Val Val Val Gly Gly
            180                 185                 190

Val Leu Ala Cys Tyr Ser Leu Leu Val Thr Val Ala Phe Ile Ile Phe
        195                 200                 205

Trp Val Arg Ser Lys Arg Ser Arg Gly Gly Gly Gly Ser Lys Arg Gly
    210                 215                 220

Arg Lys Lys Leu Leu Tyr Ile Phe Lys Gln Pro Phe Met Arg Pro Val
225                 230                 235                 240

Gln Thr Thr Gln Glu Glu Asp Gly Cys Ser Cys Arg Phe Pro Glu Glu
                245                 250                 255

Glu Glu Gly Gly Cys Glu Leu Gly Gly Gly Gly Ser Gly Gly Gly Ala
            260                 265                 270

Ser Gly Gly Gly Gly Ser Phe Ser Arg Ser Ala Asp Ala Pro Ala Tyr
        275                 280                 285

Gln Gln Gly Gln Asn Gln Leu Tyr Asn Glu Leu Asn Leu Gly Arg Arg
    290                 295                 300

Glu Glu Tyr Asp Val Leu Asp Lys Arg Arg Gly Arg Asp Pro Glu Met
305                 310                 315                 320

Gly Gly Lys Pro Arg Arg Lys Asn Pro Gln Glu Gly Leu Tyr Asn Glu
                325                 330                 335

Leu Gln Lys Asp Lys Met Ala Glu Ala Tyr Ser Glu Ile Gly Met Lys
            340                 345                 350

Gly Glu Arg Arg Arg Gly Lys Gly His Asp Gly Leu Tyr Gln Gly Leu
        355                 360                 365

Ser Thr Ala Thr Lys Asp Thr Tyr Gly Ala Leu His Met Gln Ala Leu
    370                 375                 380

Pro Pro Arg
385

<210>  28
<211>  10
<212>  PRT
<213>  Homo sapiens

<400>  28

Lys Pro Leu Pro Glu Val Thr Asp Glu Tyr
1               5                   10


<210>  29
<211>  17
<212>  PRT
<213>  Homo sapiens

<400>  29

Glu Lys Glu Ala Leu Lys Lys Ile Ile Glu Asp Gln Gln Glu Ser Leu
1               5                   10                  15

Asn


<210>  30
<211>  120
<212>  PRT
<213>  Artificial Sequence

<220>
<223>  Mouse human chimera

<400>  30

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr His Tyr
            20                  25                  30

Tyr Ile Tyr Trp Val Arg Gln Ala Pro Gly Gln Gly Leu Glu Trp Met
        35                  40                  45

Gly Gly Val Asn Pro Ser Asn Gly Gly Thr His Phe Asn Glu Lys Phe
    50                  55                  60

Lys Ser Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Glu Tyr Asp Tyr Gly Leu Gly Phe Ala Tyr Trp Gly Gln
            100                 105                 110

Gly Thr Leu Val Thr Val Ser Ser
        115                 120

<210>  31
<211>  112
<212>  PRT
<213>  Artificial Sequence

<220>
<223>  Mouse human chimera

<400>  31

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Pro Lys Asn Tyr Leu Ala Trp Tyr Gln Gln Lys Pro Gly Gln
        35                  40                  45

Pro Pro Lys Leu Leu Ile Tyr Trp Ala Ser Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Tyr Asn Leu Leu Thr Phe Gly Gly Gly Thr Lys Val Glu Ile Lys
            100                 105                 110

<210>  32
<211>  10
<212>  PRT
<213>  Homo sapiens

<400>  32

Glu Gln Lys Leu Ile Ser Glu Glu Asp Leu
1               5                   10

<210>  33
<211>  6
<212>  PRT
<213>  Homo sapiens

<400>  33

His His His His His His
1               5

<210>  34
<211>  112
<212>  PRT
<213>  Artificial Sequence

<220>
<223>  Mouse human chimera

<400>  34

Asp Ile Val Met Thr Gln Ser Pro Asp Ser Leu Ala Val Ser Leu Gly
1               5                   10                  15

Glu Arg Ala Thr Ile Asn Cys Arg Ser Ser Gln Ser Leu Leu Asp Ser
            20                  25                  30

Arg Thr Lys Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Tyr Asn Leu Pro Thr Phe Gly Gly Gly Thr Lys Val Glu Ile Lys
            100                 105                 110


<210>  35
<211>  118
<212>  PRT
<213>  Artificial Sequence

<220>
<223>  Mouse human chimera

<400>  35

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Trp Met Asn Trp Val Arg Gln Ala Pro Gly Lys Gly Leu Glu Trp Val
        35                  40                  45

Gly Gln Ile Arg Asn Lys Pro Asn Asn Tyr Glu Thr Tyr Tyr Ser Asp

50                  55                  60

Ser Leu Lys Gly Arg Phe Thr Ile Ser Arg Asp Asp Ser Lys Ser Ile
65                  70                  75                  80

Thr Tyr Leu Gln Met Asn Ser Leu Arg Ala Glu Asp Thr Ala Val Tyr
                85                  90                  95

Tyr Cys Thr Leu Gly Asn Ser Trp Phe Ala Tyr Trp Gly Gln Gly Thr
            100                 105                 110

Leu Val Thr Val Ser Ser
        115

<210>  36
<211>  115
<212>  PRT
<213>  Artificial Sequence

<220>
<223>  Human mouse chimera

<400>  36

Glu Val Gln Leu Gln Gln Ser Gly Pro Glu Leu Val Lys Pro Gly Thr
1               5                   10                  15

Ser Val Arg Ile Ser Cys Lys Thr Ser Gly Tyr Thr Phe Thr Glu Tyr
            20                  25                  30

Thr Ile His Trp Val Lys Gln Ser His Gly Lys Ser Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Ala Gly Trp Asn Phe Asp Tyr Trp Gly Gln Gly Thr Thr Val Thr
            100                 105                 110

Val Ser Ser
        115

<210>  37
<211>  107

<212>  PRT
<213>  Artificial Sequence

<220>
<223>  Human mouse chimera

<400>  37

Asp Ile Val Met Thr Gln Ser His Lys Phe Met Ser Thr Ser Val Gly
1               5                   10                  15

Asp Arg Val Ser Ile Ile Cys Lys Ala Ser Gln Asp Val Gly Thr Ala
            20                  25                  30

Val Asp Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Phe Thr Leu Thr Ile Thr Asn Val Gln Ser
65                  70                  75                  80

Glu Asp Leu Ala Asp Tyr Phe Cys Gln Gln Tyr Asn Ser Tyr Pro Leu
                85                  90                  95

Thr Phe Gly Ala Gly Thr Met Leu Asp Leu Lys
            100                 105


<210>  38
<211>  119
<212>  PRT
<213>  Artificial Sequence

<220>
<223>  Human mouse chimera

<400>  38

Glu Val Gln Leu Gln Gln Ser Gly Pro Glu Leu Val Lys Pro Gly Ala
1               5                   10                  15

Ser Val Lys Met Ser Cys Lys Ala Ser Gly Tyr Thr Phe Thr Asp Tyr
            20                  25                  30

Tyr Met Lys Trp Val Lys Gln Ser His Gly Lys Ser Leu Glu Trp Ile
        35                  40                  45

Gly Asp Ile Ile Pro Ser Asn Gly Ala Thr Phe Tyr Asn Gln Lys Phe
    50                  55                  60

Lys Gly Lys Ala Thr Leu Thr Val Asp Arg Ser Ser Ser Thr Ala Tyr

65                  70                  75                  80

Met His Leu Asn Ser Leu Thr Ser Glu Asp Ser Ala Val Tyr Tyr Cys
                85                  90                  95

Thr Arg Ser His Leu Leu Arg Ala Ser Trp Phe Ala Tyr Trp Gly Gln
            100                 105                 110

Gly Thr Leu Val Thr Val Ser
        115

<210>  39
<211>  113
<212>  PRT
<213>  Artificial Sequence

<220>
<223>  Human mouse chimera

<400>  39

Asp Phe Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Leu Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Tyr Thr Phe Gly Gly Gly Thr Lys Leu Glu Ile
            100                 105                 110

Lys

## Description

The present invention relates to immune cell-based therapeutics and methods of using the therapeutics in the treatment of cancer, infections and autoimmune disorders.

Chimeric antigen receptors (CARs) are artificial receptors consisting of a binding moiety, which provides the antigen-specificity and one or several signaling chains derived from immune receptors (Cartellieri et al., J.Biomed.Biotechnol. doi: 10.1155/2010/956304 (2010)). These two principal CAR domains are connected by a linking peptide chain including a transmembrane domain, which anchors the CAR in the cellular plasma membrane. Immune cells, in particular T and NK lymphocytes, can be genetically modified to express CARs inserted into their plasma membrane. If such a CAR modified immune cell encounters other cells or tissue structures expressing or being decorated with the appropriate target of the CAR binding moiety, upon binding of the CAR binding moiety to the target antigen the CAR modified immune cell is crosslinked to the target. Cross-linking leads to an induction of signal pathways via the CAR signaling chains, which will change the biologic properties of the CAR engrafted immune cell. For example, CAR triggering in effector CD4+ and CD8+ T cells will activate typical effector functions like secretion of lytic compounds and cytokines which will eventually lead to the killing of the respective target cell. The adoptive transfer of immune cells engineered with chimeric antigen receptors (CARs) is currently considered as a highly promising therapeutic option for treatment of otherwise incurable malignant, infectious or autoimmune diseases. Until today, two CAR-T cell therapeutics have gained market approval for treatment of B cell derived malignancies, proving the clinical feasibility of this approach.

However, the conventional CAR technology comes along with a number of critical issues which need to be solved before this treatment modality can be widely applied for clinical treatments. First of all, several safety issues have to be addressed. So far, immune responses of T cells engineered with conventional CARs are difficult to control after infusion into the patient Especially unexpected target gene expression on healthy tissue may provoke a rapid and rigorous immune reaction of engineered T cells against healthy cells, which can cause severe side effects (Lamers et al. J.Clin.Oncol. 24 e20 - e22 (2006), Morgan et al. Mol.Ther. 18: p.843 - 851 (2010). Organ damage could also be induced by unexpected cross-reactivity of the CAR binding domain with antigens expressed on healthy tissue. Although this has not been reported for CAR-T cells until now, as many trials are in an early stage, it has been observed in clinical trials with T cells genetically engineered to express recombinant T cell receptors (Linette et al, Blood 2013, Morgan et al. J. Immunother. 2013). Moreover, as CAR-T cells are a new class of self-amplifying cell drugs, infused T cells can undergo a vigorous expansion in the presenece of heavy tumor burden leading to tumor lysis syndrome and cytokine release syndrome (Brudno and Kochenderfer, Blood 2016; Maude et al. Cancer J. 2014). Another drawback of conventional CAR technology is the restriction of engineered T cell retargeting to a single antigen. Such a monotherapeutic approach implies the risk for development of tumor escape variants, which have lost the target antigen during treatment. The emergence of tumor escape variants under conventional CAR T cell therapy after several months was already observed in clinical trials, (Grupp et al. N.Engl.J.Med. 368: 1509 - 1518 (2013)). Taken together, these obstacles restrict the application of CAR-T cells to very few indications. In fact, examples of clinical effectiveness have been restricted to CD19+ CAR-T cells until now.

Modular "universal" CAR-T (UniCAR) approaches can overcome these limitations by separating antigen-recognition and activating domain of a CAR into two separate operational units. T cells are engineered to express a CAR with a universal binding domain recognizing a tag (Cartellieri et al. Blood cancer J. 2016; Rodgers et al. 2016 PNAS). Antigen-specificty is provided by soluble adapter proteins, which consist of an antigen-binding domain fused to the tag recognized by the universal CAR.

WO 2012082841 A2 discloses universal anti-tag chimeric antigen receptor-expressing T cells and methods of treating cell related disorders, e.g. cancer.

In addition WO 2013044225 A1 discloses a universal immune receptor expressed byT cells for the targeting of diverse and multiple antigens.

Both methods describe the use of modified T cells expressing universal anti-tag immune receptors. These T cells can be redirected to disease-related cell surface antigens by additionally applying modules binding these surface antigens and carrying the respective tag. The problem arising from the aforesaid methods is that a redirection of the genetically modified T cells using exogenous tags is likely to be immunogenic, which will put patients in danger and negatively affect efficacy of treatment.

WO 2016030414 A1 provides a genetically modified immune cell that allows a redirection against diverse disorders in a safe and efficient manner using endogenous tags based on nuclear proteins.

The binding moieties of these UniCAR T cell are however still single-chain fragment variables (scFv) and thus are active binding domains. Such an active binding domain has several disadvantages. First, it still has the risk of an on target/off tumor activity in case the target tag is present on healthy tissue. Secondly, there is a theroretical risk for cross-reactivity of the binding domain towards non-target cell surface proteins leading to off tumor activation and could potentially induce severe tissue damage. Finally, the coding sequence of an scFv is rather long, which is a disadvantage for manufacturing genetically engineered cells.

Therefore, it is an object of the present invention to provide a genetically modified immune cell that allows a redirection against diverse disorders in a safe and efficient manner using a small cell surface binding moiety, which would minimize the risk for cross-reactivity of the CAR-modified immune cell and significantly shorten the coding sequence allowing for genetic modification of an immune cell with several CAR constructs. It is a further object of the present invention to provide a method of treatment of diverse cell related disorders, wherein the length and intensity of treatment is adjustable in a simple mannerto the clinical demand.

The present invention provides a reversed universal, modularchimeric antigen receptor (RevCAR) system that allows a retargeting of RevCAR engrafted immune cells against multiple antigens. The system uses a gene therapy platform to generate immune cells capable of recognizing various antigens and that have broad and valuable clinical implications for the use of immune cell-based therapies, in particular T- and NK-cell based therapies.

In a first aspect, the present invention provides an isolated nucleic acid sequence encoding a reversed universal chimeric antigen receptor, wherein the receptor comprises three domains, wherein the first domain is a peptide epitope tag serving as cell surface binding domain, the second domain is a linking peptide chain including an extracellular hinge and a transmembrane domain and the third domain is a signal transduction domain, wherein the peptide epitope tag serving as cell surface binding domain is a linear epitope. Particularly suitable cell surface peptide epitope tags are human peptide sequences, especially peptide sequences derived from human nuclear proteins (i.e. the La protein), most especially peptide sequences known not to be the target of autoantibodies in autoimmune patients (i.e. the 5B9 epitope of the La protein), thus making it unlikely that the tag is immunogenic in the context of the reversed universal chimeric receptor. An optional fourth domain is a short peptide linker in the extracellular portion of RevCARs, which forms a linear epitope for a monoclonal antibody (mab) specifically binding to the fourth domain. This additional domain is not required for functionality of the RevCAR system but may add additional clinical benefit to the invention. Preferably, the present invention provides an isolated nucleic acid sequence encoding a reversed universal chimeric antigen receptor according to the present invention, wherein the nucleic acid sequence encodes for an artificial chimeric fusion protein and wherein the nucleic acid sequence is provided as cDNA.

In a further aspect the present invention provides an adapter module (AdMo) composed of a binding moiety specific for a certain human cell surface protein or protein complex and a binding moiety specific for the peptide epitope tag serving as cell surface binding domain of the RevCAR.

In a further aspect the present invention provides a nucleic acid encoding an adapter module according to the present invention. Preferably the present invention provides an isolated nucleic acid sequence encoding an adapter module according to the present invention, wherein the isolated nucleic acid is provided as cDNA.

In a further aspect the present invention provides a cell comprising a nucleic acid encoding a reversed universal chimeric antigen receptor according to the present invention comprising three domains, wherein the first domain is a peptide epitope tag serving as cell surface binding domain, the second domain is a linking peptide chain including an extracellular hinge and a transmembrane domain and the third domain is a signal transduction domain.

In a further aspect the present invention provides a cell comprising nucleic acids encoding two or more reversed universal chimeric antigen receptors according to the present invention each comprising three domains, wherein the first domain is a peptide epitope tag serving as cell surface binding domain, the second domain is a linking peptide chain including an extracellular hinge and a transmembrane domain and the third domain is a signal transduction domain.

In a further aspect the present invention provides a vector comprising a nucleic acid encoding a reversed universal chimeric antigen receptor according to the present invention, wherein the reversed universal chimeric antigen receptor comprises three domains, wherein the first domain is a peptide epitope tag serving as cell surface binding domain, the second domain is a linking peptide chain including an extracellular hinge and a transmembrane domain and the third domain is a signal transduction domain.

In a further aspect the present invention provides a kit comprising a vector according to the present invention comprising a nucleic acid sequence encoding a reversed universal chimeric antigen receptor according to the present invention and an adapter moduleaccording to the present invention and/or a vector encoding an isolated nucleic acid sequence encoding an adapter moduleaccording to the present invention.

The invention encompasses moreover a pharmaceutical composition that contains cells and adapter modulesaccording to the invention in association with a pharmaceutically acceptable dilution agent or carrier. Preferably, the pharmaceutical composition is present in a form suitable for intravenous administration.

Preferably, the composition comprises cells comprising a nucleic acid encoding a reversed universal chimeric antigen receptor according to the present invention and adapter modules according to the present invention.

The pharmaceutical composition according to the invention comprises various administration forms. The pharmaceutical compositions are preferably administered parenterally, particularly preferred intravenously. In one embodiment of the invention, the parenteral pharmaceutical composition exists in an administration form that is suitable for injection. Particularly preferred compositions are therefore solutions, emulsions, or suspensions of the cell and adapter modulethat are present in a pharmaceutically acceptable dilution agent or carrier.

As a carrier, preferably water, buffered water, 0.9 % saline solution, glycine solution and similar solvents are used. The solutions are sterile. The pharmaceutical compositions are sterilized by conventional well-known techniques. The compositions contain preferably pharmaceutically acceptable excipients, for example, those that are required in order to provide approximately physiological conditions and/or to increase the stability of the adapter modules, such as agents for adjusting the pH value and buffering agents, preferably selected from sodium acetate, sodium chloride, sodium citrate, potassium phosphate, potassium chloride, calcium chloride, sodium lactate and histidine. The concentrations of adapter modules according to the invention in these formulations, depending on the application, are variable; they are preferably less than 0.01 % by weight, preferably at least 0.1 % by weight, further preferred between 1 and 5 % by weight and they are selected primarily on the basis of fluid volumes, viscosity etc. or in compliance with the respective administration mode.

Pharmaceutical compositions must be sterile and stable under the manufacturing and storage conditions. The composition can be formulated as a solution, microemulsion, dispersion, in liposomes or in other ordered structures that are suitable for this purpose and known by the artesian.

The cells and adapter modules according to the invention are preferably introduced into a composition that is suitable for parenteral administration. Preferably, the pharmaceutical composition is an injectable buffered solution that contains between 1 ng/mL to 500 mg/ml of AdMo, especially preferred between 5 ng/mL to 250 mg/ml of adapter module, in particular together with 1 to 500 mmol/l (mM) of a buffer, especially preferred 5 to 20 mM. The injectable solution can be present in liquid form. The buffer can be preferably histidine (preferably 1 to 50 mM, especially preferred 5 to 20 mM) at a pH value of 5.0 to 7.0 (especially preferred at a pH of 6.0).

Other suitable buffers encompass, but are explicitly not limited to, sodium succinate, sodium citrate, sodium phosphate, or potassium phosphate. Preferably, sodium chloride between 0 to 300 mM, especially preferred 150 mM, is used for a liquid administration form. In liquid administration forms, stabilizers are preferably used, preferably polysorbate-80 between 0.0001% (w/v) and 1% (w/v), especially preferred between 0.001% (w/v) and 0.1% (w/v).

A typical dose-rate delivered per patient per day is between 1 ng to 1000 mg, preferably 3 ng to 3 mg, with dosages administered one or more times per day or week or continuously over a period of up to several weeks.

In a further aspect the invention provides the use of cells according to the present invention comprising a nucleic acid encoding a universal chimeric antigen receptor according to the present invention and adapter modulesaccording to the present invention for stimulating a universal chimeric antigen receptor mediated immune response in mammals. Preferably the invention provides the use of cells according to the present invention comprising a nucleic acid encoding a reversed universal chimeric antigen receptor according to the present invention and adapter modules according to the present invention as a medication, more preferably as a medication for treatment of cancer or an autoimmune disease. An autoimmune disease arises from an abnormal immune response of the body against substances and tissues normally present in the body (autoimmunity).

The invention comprises further the use of cells and adapter modules according to the invention for preparing a medication for therapeutic and/or diagnostic use in case of cancer or an autoimmune disease.

The invention also encompasses a method for treatment of a human having cancer, infectious, inflammatory or an autoimmune disease by administration of cells and adapter modules according to the invention.

For therapeutic applications, a sterile pharmaceutical composition, containing a pharmacologically effective quantity of cells and adapter modules according to the invention, is administered to a patient in order to treat the aforementioned illnesses.

The invention will be explained in more detail with the aid of the following figures and embodiments without limiting the invention to them.
Fig. 1 depicts a schematic illustration of the reversed universal chimeric antigen receptor (RevCAR). LP, leader peptide for translation into the endplasmatic reticulum and transport to the cell membrane; PE, peptide epitope as passive binding domain of the RevCAR; PL; optional fourth domain for recognition and/or purification; ECD, extracellular domain as hinge domain; TM, transmembrane domain; ICD 1 and ICD 2, intracellular signaling domain, which can consist of a single domain or two or multiple domains.
Fig. 2 shows a schematic illustration of the reversed universal chimeric antigen receptor (RevCAR) platform for antigen-specific immune cell retargeting. In the example, the immune cell is a T cell (T). AdMo, adapter molecule, constructed from two single-chain fragment variables in the example; PE, peptide epitope as passive binding domain of the RevCAR; RevCAR, reversed universal chimeric antigen receptor; TCR, endogenous T cell receptor.
Fig. 3 depicts a schematic drawing of two adapter modules, which both specifically bind to CD123, an antigen commonly expressed on leukemias. The adapter molecules are constructed in a way, that they can either recruit RevCARs harboring a La5B9 epitope tag (AdMo CD123-La5B9) or harboring a La7B6 tag (AdMo CD123-La7B6).
Fig. 4 shows the affinity of two CD123-specific adapter modules towards target antigen CD123 as determined by binding assay on CD123-expressing OCI-AML3 blasts (A) and towards their RevCAR tag, which is either the La 5B9 or the La 7B6 tag, respectively (B).
Fig. 5 shows specific lysis of CD123-positive AML blasts by human primary T cells genetically engineered to express either a RevCAR harboring a La5B9 tag (A) or a La7B6 tag (B). Specific lysis is induced in a concentration-dependent manner in the presence of the corresponding CD123-specific adapter modules recognizing either the La5B9 tag (AdMo CD123-La5B9) or La7B6 tag (AdMo CD123-La7B6).
Fig. 6 shows a schematic map of the lentiviral vector pLVX-EF1α-IRES-ZsGreen1.
Fig. 7 shows a schematic map of the lentiviral packaging plasmid psPAX2.
Fig. 8 shows a schematic map of the envelope plasmid pMD2.G.

Tab. 1 summarizes the molecule numbers per cell for RevCAR constructs 1-4 on the cell surface of lentivirally transduced T cells. Molecule numbers were quantified using the QIFIKIT (Agilent, Santa Clara, CA, USA) with the monoclonal anti-La antibodies 5B9 or 7B6 respectively.

### Effector cells

The effector cells used in the methods of the present invention may be autologous, syngeneic or allogeneic, with the selection dependent on the disease to be treated and the rreans available to do so. Suitable populations of effector cells that may be used in the methods include any immune cells with cytolytic, phagocytic or immunosuppressive activity, such as T cells, including regulatory T cells, NK cells and macrophages. In one aspect, effector cells are from a certain HLA background and utilized in an autologous or allogeneic system. Effector cells can be isolated from any source, including from a tumor explant of the subject being treated or intratumoral cells of the subject being treated. In an embodiment, effector cells may be generated by *in vitro* differentiation from pluri- or multipotent stem or progenitor cells prior to or after genetic manipulation of the respective cells to express RevCARs. In the following, the term "effector cell" refers to any kind of aforementioned immune cells genetically altered to express RevCARs on their cell surface.

### Reversed universal chimeric antigen receptor (RevCAR)

The RevCAR expressed by effector cells used in the methods of the present invention allows for a modular, highly flexible and tightly controllable retargeting of RevCAR expressing immune cells in an antigen-specific manner. The sole requirements for the RevCARs used in the methods are (i) that the RevCAR via its cell surface peptide epitope tag has binding specificity for a particular tag binding moiety that can be conjugated to a adapter module, which in turn binds to a cellular surface protein or an extracellular structure of a target cell, and (ii) that immune cells can be engineered to express RevCAR.

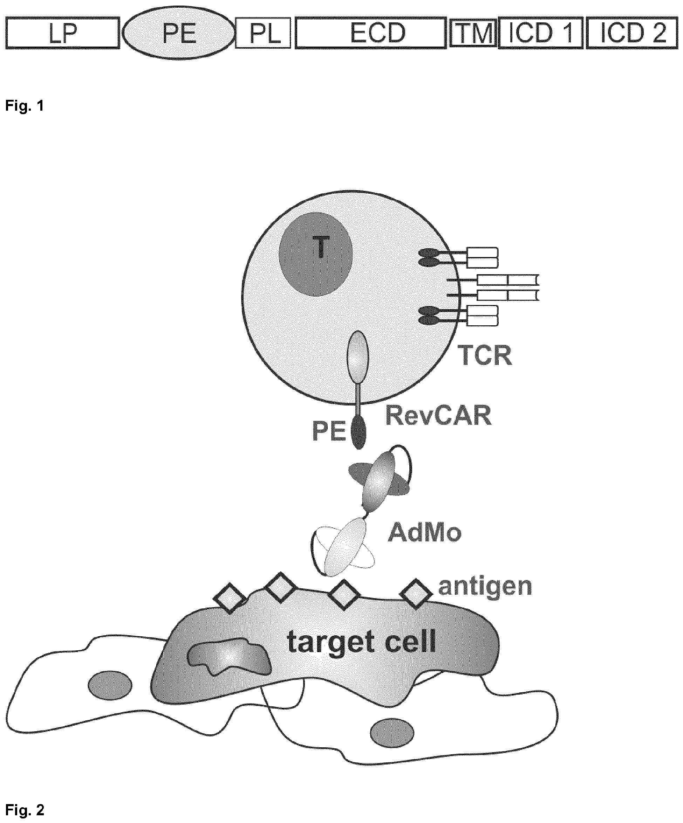

The RevCAR comprises three domains Fig. 1. The first domain is the cell surface peptide epitope tag. This cell surface peptide epitope tag is typically present at the amino terminal end of the polypeptide that comprises the RevCAR. Locating the cell surface peptide epitope tag at the amino terminus permits the cell surface peptide epitope tag unhampered access to an adapter module that is bound to a target cell. The cell surface peptide epitope tag is typically a linear peptide epitope.

In a preferred embodiment the cell surface peptide epitope tag is a short linear peptide epitope derived from a human protein.

In a more preferred embodiment the cell surface peptide epitope tag is a short linear peptide epitope derived from a human nuclear protein.

In the most preferred embodiment the cell surface peptide epitope tag is a short linear peptide epitope derived from the human nuclear La protein. Preferably the tag is selected from human La epitopes E5B9 or E7B6. The use of the E5B9 and E7B6 La epitopes in the RevCAR system is advantageous due to the fact that the anti-E5B9 and anti-E7B6 scFvs do not interact with native La protein bound to the surface of cells under normal physiological conditions and no aut-antibodies against these epitopes have been observed in auto-immune patients who are reported to often have auto-antibodies against the La protein.

The second domain of the RevCAR is an extracellular hinge and a transmembrane (TM) domain. The hinge domain allows the RevCAR to protrude from the surface of the effector cell for optimal binding to its corresponding scFv. The TM domain anchors the RevCAR into the cell membrane of the effector cell. Exemplary hinge and TM domains include, but are not limited to, the hinge and transmembrane regions of the human CD28 molecule, the CD8a chain, NK cell receptors like natural killer group 2D (NKG2D), DAP12 or parts of the constant region of an antibody as well as combinations of various hinge and TM domains.

The third domain, when present, is the signal transduction domain. This domain transmits a cellular signal into the RevCAR carrying effector cell upon cross-linkage of the effector cell to a cell or extracellular structure. Cross-linkage between effector and target cell is mediated and depends on the presence of (i) an adapter module which binds to its particular binding moiety on the target cell or target extracellular structure and carries a RevCAR binding moiety and (ii) the RevCAR expressed on the surface of the effector cell presenting the peptide epitope tag that can be recognized and bound by the adapter module. Effector cell activation includes induction of cytokines or chemokines as well as activation of cytolytic, phagocytic or suppressive activity of the effector cell. Exemplary effector cell signal transduction domains include, but are not limited to, the cytoplasmic regions of CD28, CD137 (41BB), CD134 (OX40), DAP10 and CD27, which serve to enhance T cell survival and proliferation; inhibitory receptors as programmed cell death-1 (PD-1) and cytotoxic T-lymphocyte antigen 4 (CTLA-4) as well as cytoplasmic regions of the CD3 chains (e.g. CD3zeta), DAP12 and Fc receptors, which induce T and NK cell activation. One or more than one signal transduction domains may be included in the RevCAR, such as two, three, four or more immune cell activating or costimulatory domains.

In a further embodiment the RevCAR comprises a fourth domain, which is a short peptide linker in the extracellular portion of the RevCAR (Fig. 1). It is required for its functionality, that this fourth domain forms a linear epitope which allows the binding of a specific monoclonal antibody with reasonable affinity. One or more than one linear epitope may be included in the fourth domain and they may be located as linker in the tag-binding domain, in between the tag-binding domain and the extracellular linker or may be an integral part of the extracellular hinge domain. With the help of the optional fourth domain RevCAR engrafted immune cells can be specifically stimulated, so that RevCAR engrafted immune cells proliferate preferentially and persist longer compared to non-engrafted immune cells either *in vitro* or *in vivo.* The fourth domain may be also used to purify RevCAR engrafted immune cells from mixed cell populations. It may be also used to dampen RevCAR engrafted immune cell mediated immune response and to eliminate RevCAR engrafted immune cells *in vivo.*

To allow for expression on the cell surface of an effector cell, a signal peptide (sometimes also referred to as signal sequence, targeting signal, or leader peptide) is put in front of the peptide epitope serving as extracellular binding domain at the 5' end of the RevCAR nucleic acid sequence coding for its N-terminus. Signal peptides target proteins to the secretory pathway either co-translationally or post-translationally. For this purpose signal peptides from proteins of various species can be utilized, however preferentially leader peptides from proteins like CD28, CD8alpha, IL-2 or the heavy or light chain of antibodies of human origin are used to avoid immunogenic reactions.

### Adapter modules

Adapter modules are composed of a binding moiety specific for a certain human cell surface protein or protein complex and a binding moiety, which binds to the cell surface peptide epitope of RevCAR receptors according to the present invention. Adapter modules are administered to a subject prior to, or concurrent with, or after administration of the RevCAR-expressing effector cells. Alternatively, RevCAR expressing effector cells may be decorated with adapter modules prior to the infusion into the recipient. Potential binding moieties of adapter modules include, but are not limited to, antibodies or fragments thereof that bind to surface antigens like CD2, CD3, CD4, CD8, CD10, CD19, CD20, CD22, CD25, CD23, CD30, CD33, CD38, CD44, CD52, CD90, CD99, CD123, CD223, CD269, CD274, CD276, CD279 and CD366, members of the epidermal growth factor receptor family (ErbB1, ErbB2, ErbB3, ErbB4 and mutants thereof), epidermal growth factor receptor (EGFR), members of the ephrin receptor family (EphA1-10, EphB1-6), so called prostate specific antigens (e.g. prostate stem cell antigen PSCA, prostate specific membrane antigen PSMA), embryonic antigens (e.g. carcinoembryonic antigen CEA, fetal acethylcholine receptor), members of the vascular endothelia growth factor family (VEGFR 1-3), epithelia cell adhesion molecule EpCAM, alphafetoprotein AFP, members of the mucin protein family (e.g. MUC1, MUC16), follicle stimulating hormone receptor (FSHR), the human high molecular weight-melanoma-associated antigen (HMW-MAA), folate binding protein FBP, a-Folate receptor, ligands of the NKG2D receptor, cytokine receptors [e.g. IL-8Rα (CXCR1), IL-8Rβ (CXCR2), IL-11Rα, IL-11Rβ, IL-13Rα1 and 2, CXCR4], members of the epithelia glycoprotein family (e.g. EGP-2, EGP-4), diasialogangliosides (e.g. GD2, GD3), members of the carbonic anhydrase family (e.g. CAIX), members of the carbohydrate antigen family (e.g. Ley), Notch ligands (e.g. Delta-like 1 and 4), melanoma-associated chondroitin sulfate proteoglycan (MCSP), glycoprotein A33, and tumor-specific glycans [e.g. serine- or threonine-linked N-acetylgalactosamine (Tn) or derivatives like sialyl-Tn], including mutants of the named proteins and protein families. In addition, the binding moiety of adapter modules include, but are not limited to, antibodies or fragments thereof that binds to cytoplasmic or nuclear antigens like the La/SSB antigen, members of the Rho family of GTPases, members of the high mobility group proteins and others. Likewise, the binding moiety of a adapter module can be composed of the alpha and beta or the gamma and delta chains of a T cell receptor (TCR) or fragments thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g. autoimmunogenic cancer/testis antigen NY-ESO-1, members of the melanoma antigen family A (MAGEA), the preferentially expressed antigen in melanoma (PRAME), and leukemia-associated antigens (e.g. wilms tumor gene 1 WT1). The binding moiety of adapter modules can also comprise ligands to proteins and protein complexes, further on referred as receptors, or fragments thereof. Such ligands may bind to, but are not limited to, cytokine receptors (e.g. IL-13 receptor), ligands of the NKG2D receptor, ligands to the EGFR family members, ligands of checkpoints molecules like PD-1, CTLA-4, lymphocyte-activation gene 3 (LAG-3) or T-cell immunoglobulin and mucin-domain containing-3 (TIM-3), or auto-reactive TCRs. Moreover, target binding moieties could also be chemically synthesized peptide derivatives that are fused to the tag binding moiety via a chemical reaction (i.e. click chemistry). In a preferred embodiment, peptides with binding specificity to membrane receptors have a binding specificity to membrane receptors selected from CD2, CD3, CD4, CD8, CD10, CD19, CD20, CD22, CD25, CD23, CD30, CD33, CD38, CD44, CD52, CD90, CD99, CD123, CD223, CD269, CD274, CD276, CD279 and CD366, interleukin receptors, especially preferred IL-8Rα (CXCR1), IL-8Rβ (CXCR2), IL-11Rα, IL-11Rβ, IL-13Rα1 and 2, CXCR4; c-Met, transforming growth factor β receptors, erbB1, erbB2, erbB3, erbB4 and mutants thereof, ephrin receptors, especially preferred EphA1-10, EphA5 or EphB1-6; prostate stem cell antigen (PSCA), prostate specific membrane antigen (PSMA), carcinoembryonic antigen (CEA), fetal acetylcholine receptor, onco-fetal antigens, tumor-specific glycans [e.g. serine- or threonine-linked N-acetylgalactosamine (Tn) or derivatives like sialyl-Tn]; VEGFR 1, VEGFR 2 or VEGFR 3, Neuropilin-1, epithelia cell adhesion molecule (EpCAM), epidermal growth factor receptor (EGFR), alphafetoprotein (AFP), mucins, especially preferred MUC1, MUC16 or MUC18; follicle stimulating hormone receptor (FSHR), human high molecular weight-melanoma-associated antigen (HMW-MAA), folate binding protein (FBP), a folate receptor, NKG2D, major histocompatibility complex (MHC) class I molecules, especially preferred MHC class I chain-related gene A (MICA) or B (MICB), UL16 binding protein (ULPB) 1, ULPB 2, ULPB 3, ribonucleic acid export 1 (Rae-1) family members or histocompatibility 60 (H-60); chaperones and heat shock proteins, especially preferred heat shock protein (HSP) 90 or 78 kDa glucose-regulated protein (GRP78); EGP-2 or EGP-4, diasialoganglioside 2 (GD2) or GD3, carbonic anhydrase 9 (CAIX), Lewis Y (LeY), C-type lectin-like molecule-1 (CLL-1), tumor necrosis factor related apoptosis inducing ligand (TRAIL) receptor, apoptosis antigen 1 (APO-1, Fas, CD95), members of the keratin family or integrins, especially preferred avβ3 or avβ5, aminopeptidase A, aminopeptidase N or neural/glial antigen 2 (NG2).

Binding moieties of adapter modules may comprise single antigen specificity (monospecific), two, three or more antigen specificities (bi- and multispecific). Examples for bi- and multispecific antigen specificities include, but are not limited to, adapter modules binding to PSCA and PSMA antigen, CD19 and CD20 antigen, CD19, CD20, and CD22 antigen, CD33 and CD123 antigen, CD33 and CD99, CD33 and TIM-3, ErbB-1 and -2, PSCA and ErbB-2 and further combinations. Binding moieties of adapter modules may also comprise monovalent binding as well a bi- and multivalent binding sites. Examples for bi- and multivalent targeting strategies include, but are not limited to, adapter modules incorporating two scFvs recognizing different epitopes of PSCA, CEA, CD19 and CD33, and ligand-scFv combinations recognizing different epitopes of the ErbB1 receptor.

Adapter modules may also carry additional ligands, which are not involved in the target antigen binding, further on referred to as payloads. Such payloads may comprise, but are not limited to, costimulatory ligands or cytokines fused to the N- or C-terminus of the adapter module, in particular the extracellular domain of CD28, CD137 (41BB), CD134 (OX40), and CD27, as well as IL-2, IL-7, IL-12, IL-15, IL-17, and II-21, which all stimulate different kinds of immune cells. Other payloads may be radionuclides or chemical compounds which induce cell death in the target and neighboring cells.

In embodiments of the invention the cell surface peptide epitope tag of RevCAR receptors is a defined tag. The identity of the tag is only limited by the identity of the binding domain of the corresponding adapter module. The tag can be derived from any structure, against which an antibody or other binding domain is available. The antibody specific for the tag may be obtained from any species of animal, though preferably from a mammal such as human, simian, mouse, rat, rabbit, guinea pig, horse, cow, sheep, goat, pig, dog or cat. Preferably the antibodies are human or humanized antibodies. Nor is there a limitation on the particular class of antibody that may be used, including IgGI, IgG2, IgG3, IgG4, IgM, IgA1, IgA2, IgD and IgE antibodies. Antibody fragments include single-chain variable fragment (scFv), single chain antibodies, F(ab')2 fragments, Fab fragments, and fragments produced by a Fab expression library, with the only limitation being that the antibody fragments retain the ability to bind the selected tag. The antibodies may also be polyclonal, monoclonal, or chimeric antibodies, such as where an antigen binding region (e.g. F(ab')2 or hypervariable region) of a non-human antibody is transferred into the framework of a human antibody by recombinant DNA techniques to produce a substantially human molecule. Antigen-binding fragments, such as scFv, may be prepared therefrom. Antibodies to a selected tag may be produced by immunization of various hosts including, but not limited to, goats, rabbits, rats, mice, humans, through injection with a particular protein or any portion, fragment or oligopeptide that retains immunogenic properties of the protein.

Depending on the host species, various adjuvants can be used to increase the immunological response. Such adjuvants include, but are not limited to, detoxified heat labile toxin from E. coli, Freund's, mineral gels such as aluminum hydroxide, and surface-active substances such as lysolecithin, pluronic polyols, polyanions, peptides, oil emulsions, keyhole limpet hemocyanin, and dinitrophenol. BCG (Bacillus Calmette-Guerin) and Corynebacterium parvum are also potentially useful adjuvants. Antibodies and fragments thereof can be prepared using any technique that provides for the production of antibody molecules, such as by continuous cell lines in culture for monoclonal antibody production. Such techniques include, but are not limited to, the hybridoma technique originally described by Koehler and Milstein (Nature 256:495-497 (1975)), the human B-cell hybridoma technique (Kosbor et al., Immunol Today 4:72 (1983); Cote et al., Proc Natl. Acad. Sci 80:2026-2030 (1983)), and the EBV-hybridoma technique (Cole et al., Monoclonal Antibodies and Cancer Therapy, Alan R. Liss Inc, New York N.Y., pp 77-96 (1985)). Techniques developed for the production of "chimeric antibodies", i.e., the splicing of mouse antibody genes to human antibody genes to obtain a molecule with appropriate antigen specificity and biological activity can also be used (Morrison et al., Proc Natl. Acad. Sci 81:6851-6855 (1984); Neuberger et al., Nature 312:604-608 (1984); Takeda et al., Nature 314:452-454 (1985)). Alternatively, techniques described for the production of single chain antibodies can be adapted to produce tag-specific single chain antibodies.

In one aspect, the tag-binding domain is a single-chain variable fragment (scFv). A scFv comprises the variable regions of the heavy (VH) and light chains (VL) of an antibody, typically linked via a short peptide of five to about 25 amino acids. The linker can either connect the N-terminus of the VH with the C-terminus of the VL, or vice versa.

In a preferred embodiment the cell surface peptide epitope tag of the RevCAR receptor (the tag) is a short linear epitope from the human nuclear La protein. The tag-binding domain of the adapter module may constitute an antibody or an antibody-derived antigen-binding fragment, e.g. a single-chain fragment variable (scFv) binding to the respective La epitope.

Preferably, the tag is selected from human La-Epitope E5B9 or E7B6.

A method for stimulating a reversed universal chimeric antigen receptor - mediated immune response in a mammal, the method comprising:
- administering to a mammal an effective amount of an effector cell genetically modified to express one or more reversed universal chimeric antigen receptors, wherein the reversed universal chimeric antigen receptors comprise three domains, wherein the first domain is a peptide epitope tag, the second domain is an extracellular hinge and a transmembrane domain and the third domain is a signal transduction domain, wherein the tag is recognized by a binding domain suitable to generate a soluble adapor module (Fig. 2).
- administering one ore more adapter modules composed of a binding moiety specific for a certain human cell surface protein or protein complex and a binding domain, wherein the latter binding domain recognizes the cell surface peptide epitope tag of the RevCAR (Fig. 2).
wherein adapter modules are administered to a subject prior to, or concurrent with, or after administration of the reversed universal chimeric antigen receptor-expressing effector cells.

In a preferred embodiment the effector cells and adapter modules are administered to humans.

### RevCAR effector cell production

In an embodiment of the invention, immune cells may be genetically engineered to express RevCARs by various methods. In general, a polynucleotide vector encoding the RevCAR and all necessary elements to ensure its expression in the genetically engineered immune cell is transferred into the cell. The transfer of the vector can be performed, but is not limited to, by electroporation or transfection of nucleid acids or the help of viral vector systems like, but not limited to, adeno-, adeno-associated, retro-, foamy- or lentiviral viral gene transfer.

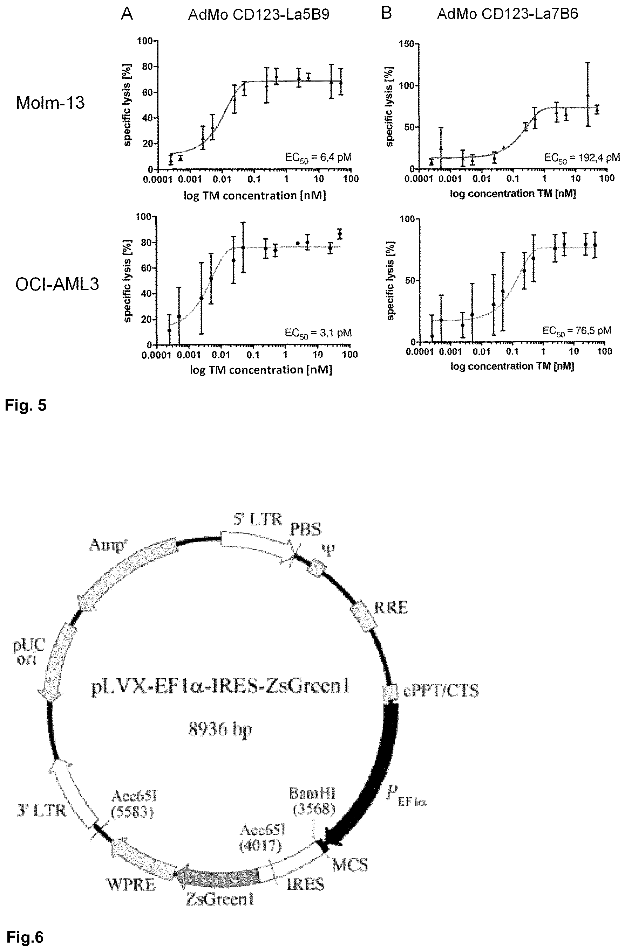

In a further embodiment, lentiviral gene transfer may be applied for stable expression of RevCARs in immune cells by first constructing a lentiviral vector encoding for a selected RevCAR. An exemplary lentiviral vector includes, but is not limited to, the vector pLVX-EF1 alpha RevCAR 28/ζ as shown in Fig. 6, in which the lentiviral parts of the vector are derived from the human immunodeficiency virus (HIV).

For the described application, the MSC/IRES/ZxGreenl portion was replaced by the RevCAR construct. Regarding Fig. 6 abbreviations are used as follows:
5' LTR: 5' long terminal repeat, PBS: primer binding site, Ψ: packaging signal, RRE: Rev-response element, cPPT/CTS: (central polypurine tract/central termination sequence, PEF1α: human elongation factor 1 alpha promoter, MCS: multiple cloning site, IRES: internal ribosome entry site, ZsGreen1: human-codon-optimized, WPRE: woodchuck hepatitis virus posttranscriptional regulatory element, 3' LTR: 3' long terminal repeat, pUC: origin of replication, Ampr: ampicillin resistance gene; β-lactamase.

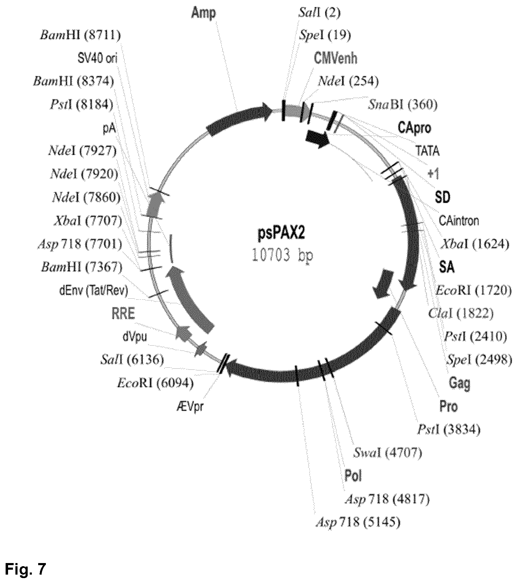

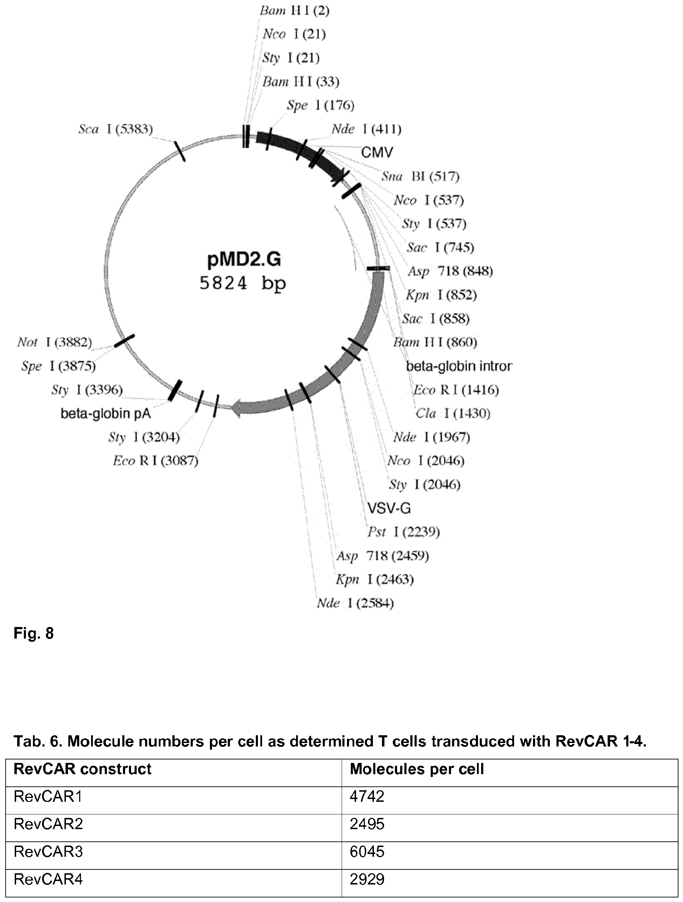

Lentiviral particles are typically produced by transient transfection of Human Embryonal Kidney (HEK) 293T (ACC 635) cells with the RevCAR encoding lentiviral vector plasmid and cotransfection with a group specific antigen (gag) and Polymerase (pol) encoding plasmid (e.g. psPAX2, addgene plasmid 12260) as depicted in Fig. 7 plus a plasmid encoding for an envelope (e.g. pMD2.G, addgene plasmid 12259) as shown in Fig. 8. After transfection the packaging plasmid expresses Gag and Pol protein of HIV-1. Abbrevation used in Fig. 7 as following: CMVenh: CMV enhancer and promoter, SD: splice donor, SA: splice acceptor, Gag: Group-specific antigen, Pro: Precursor protein encoding the protease protein, Pol: Protein encoding the reverse transcriptase and integrase, RRE: rev responsive element, Amp: ampicillin. The plasmid MD2.G (Fig. 8) encodes the glycoprotein of the Vesicular Stomatitis Virus (VSV-G). VSV-G protein is used to lentiviral vectors to transduce a broad range of mammalian cells. Abbrevation used in Fig. 8 as following: CMV: CMV enhancer and promoter, beta-globin intror: beta-globin intron, beta-globin pA: beta-globin poly adenosine tail.

Various envelopes from different virus species can be utilized for this purpose. Lentiviral vectors can successfully pseudotype, but are not limited to, with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV. Supernatants from transfected HEK293T cells can be harvested 24h to 96h after transfection and virus particles may, but not necessarily have to, be concentrated from the supernatant by ultracentrifugation or other methods. For lentiviral transduction of immune cells various established protocols can be applied. In one aspect, peripheral blood mononuclear cells (PBMC) or isolated T cells can be activated with mab specific for the CD3 complex, e.g. clone OKT3 or UCHT1, either given in solution or coated to plastic cell culture dishes or magnetic beads. Activation of PBMC or isolated T cells can be further enhanced by stimulating costimulatory pathways with mabs or ligands specific for, but not limited to, CD27, CD28, CD134 or CD137 either alone or in various combinations and the supply with exogenous recombinant cytokines like, but not limited to, interleukin (IL)-2, IL-7, IL-12, IL-15 and IL-21. Concentrated or non-concentrated virus particles are added to PBMC or T cell cultures 24h to 96h after initial administration of activating CD3 antibodies and/or recombinant cytokines as single or multiple doses. Stable transduction of T cells may be determined by flow cytometry after staining with anti-tag antibodies for surface expression of RevCARs or mabs directed against the fourth domain of RevCARs from day 3 onwards after final administration of virus supernatant. RevCAR transduced T cells can be propagated *in vitro* by culturing them under supply of recombinant cytokines and activating anti-CD3 mabs.

In a further embodiment, immune cells can be genetically engineered using gene editing techniques like transcription activator-like effector nucleases (TALEN) or clustered regularly interspaced short palindromic repeats (CRISPR/Cas) to stabily integrate the coding sequences for RevCARs into the host cell genome and to facilitate RevCAR surface expression.

In case a RevCAR harbors the optional fourth domain, a peptide sequence forming a linear epitope for a mab, immune cells genetically modified to express RevCARs can be specifically propagated *in vitro* by coating a mab or antibody fragments thereof binding to the RevCAR tag or to the optional fourth RevCAR domain to the surface of culture dishes or to beads of any kind, which are added to the cell culture at a defined ratio of, but not limited to, 1 bead to 1-4 RevCAR engrafted effector cells. The binding of surface-coated mabs to the RevCAR tag or fourth domain induces cross-linkage of cell-surface expressed RevCARs and formation of an immune synapse, which leads to the activation of signal pathways specifically triggered by the signal domain of the RevCAR. Depending on the signal pathways induced, this may leads to enhanced proliferation and sustained resistance against activation-induced cell death of the RevCAR carrying immune cells and therefore enrichment of RevCAR genetically modified immune cells in a mixed population.

The RevCAR tag or to the optional fourth domain can be further utilized to enrich and purify RevCAR expressing immune cells from mixed populations. Enrichment and purification can be performed with the help of a mab or antibody fragments thereof binding to the RevCAR tag or the fourth RevCAR domain to either mark RevCAR expressing cells for cell sorting or to transiently link the RevCAR expressing immune cell to small particles, which can be utilized for cell isolation. In one aspect, RevCAR engrafted immune cells are incubated with the mab recognizing the RevCAR tag or the fourth domain. Next, magnetic beads are added, which are conjugated with antibodies or fragments thereof directed against the species-and isotype specific heavy and light chains of the mab binding to the optional fourth domain. Thus, RevCAR expressing immune cells and magnetic beads are linked and can be trapped and separated from other immune cells in a magnetic field.

In a further embodiment of the invention the RevCAR tag or the optional fourth domain can be used for detection of UniCAR surface expression (Tab. 6). Tab. 6 depicts that RevCAR surface expression can be detected by using a monoclonal antibody directed against the RevCAR tag and subsequently staining with a fluorochrome-conjugated anti-species secondary antibody.

Populations of RevCAR-expressing immune cells may be formulated for administration to a subject using techniques known to the skilled artisan.

Formulations comprising populations of RevCAR-expressing immune cells may include pharmaceutically acceptable excipient(s). Excipients included in the formulations will have different purposes depending, for example, on the nature of the tag comprising the RevCAR, the population of immune cells used, and the mode of administration. Examples of generally used excipients include, without limitation: saline, buffered saline, dextrose, water-for- infection, glycerol, ethanol, and combinations thereof, stabilizing agents, solubilizing agents and surfactants, buffers and preservatives, tonicity agents, bulking agents, and lubricating agents. The formulations comprising populations of RevCAR-expressing immune cells will typically have been prepared and cultured in the absence of any non-human components, such as animal serum (e.g., bovine serum albumin).

A formulation may include one population or more than one, such as two, three, four, five, six or more populations of RevCAR-expressing immune cells. The different populations of RevCAR engrafted immune cells can vary based on the identity of the tag domain, the identity of the signal transduction domain, the identity of the subpopulations, the mode of generation and cultivation or a combination thereof. For example, a formulation may comprise populations of RevCAR-expressing T and NK cells that recognize and bind to one, or more than one, such as two, three, four, five, six or more different adapter modules.

The formulations comprising population(s) of RevCAR immune cells may be administered to a subject using modes and techniques known to the skilled artisan. Exemplary modes include, but are not limited to, intravenous injection. Other modes include, without limitation, intratumoral, intradermal, subcutaneous (s.c, s.q., sub-Q, Hypo), intramuscular (i.m.), intraperitoneal (i.p.), intra-arterial, intramedulary, intracardiac, intra- articular (joint), intrasynovial (joint fluid area), intracranial, intraspinal, and intrathecal (spinal fluids). Any known device useful for parenteral injection or infusion of the formulations can be used to effect such administration. Injections can be performed as bulk injections or continuous flow injections.

The formulations comprising population(s) of RevCAR-expressing immune cells that are administered to a subject comprise a number of RevCAR-expressing immune cells that is effective for the treatment and/or prophylaxis of the specific indication or disease. Thus, therapeutically-effective populations of RevCAR-expressing immune cells are administered to subjects when the methods of the present invention are practiced. The number of RevCAR-expressing immune cells administered to a subject will vary between wide limits, depending upon the location, source, identity, extent and severity of the disease, the age and condition of the individual to be treated, etc. In general, formulations are administered that comprise between about 1 x 10⁴ and about 1 x 10¹⁰ RevCAR-expressing immune cells. In most cases, the formulation will comprise between about 1 x 10⁵ and about 1 x 10⁹ RevCAR-expressing immune cells, from about 5 x 10⁵ to about 5 x 10⁸ RevCAR-expressing immune cells, or from about 1 x 10⁶ to about 1 x 10⁹ RevCAR-expressing immune cells. A physician will ultimately determine appropriate dosages to be used. In case of adverse events, RevCAR engrafted immune cells can be depleted from an individual by the administration of a mab directed against the RevCAR tag or the fourth domain, if present.

### Adapter module production

Adapter modules comprise two domains, a binding moiety specific for a certain human cell surface protein or protein complex and a tag-binding domain, which is directed against the cell surface peptide epitope tag of a RevCAR. Adapter modules can be manufactured by techniques known to the skilled artisan. These techniques include, but are not limited to, recombinant expression in pro- or eukaryotic cells, artificial synthesis of polypeptide chains or chemical synthesis.

In one aspect, an adapter module may be expressed in Chinese ovarian hamster (CHO, ACC-110) cells, which are suitable for synthesizing high amount of recombinant proteins in their biologically active forms. A nucleic acid sequence coding for an adapter module can be transferred into CHO cells by established genetically engineering techniques like, but not limited to, naked nucleic acid transfection, electroporation or viral gene transfer. High productive single-cell clones may be selected from parental lines using, for example, the dihydrofolate reductase (DHFR) selection system. In this system, DHFR-deficient CHO cell mutants (e.g. CHO sub-line DXB11 or DG44) are genetically modified by co-transfection of a functional copy of the DHFR gene in addition to the nucleic acid sequence coding for an adapter module. Clonal selection is then performed by growth in media devoid of glycine, hypoxanthine and thymidine. High-productive clones can be further selected by culturing the cells in high levels of methotrexate (MTX), a folic acid analog that blocks DHFR activity. As gene modified cells must cope with the decrease in DHFR activity, which cannot be rescued by the mere presence of a single copy of the DHFR, clones with amplified copies of the DHFR gene are favored under these conditions. The genetic linkage between DHFR and the gene of interest ensures that the transgene is also co-amplified, thus enhancing chances of securing a high producing cell clone. Selected cell clones are grown under good manufacturing conditions preferential in the absence of any animal serum. Adapter modules may be isolated from cell culture supernatants by established preparative protein purification methods including preliminary steps like precipitation or ultracentrifugation and various purification techniques like, but not limited to, size exclusion, affinity or ion exchange chromatography. In one aspect, the nucleic acid sequence of an adapter module carries a coding sequence for six to ten successive histidine amino acids which form a polyhistidine tag. The polyhistidine binds strongly to divalent metal ions such as nickel and cobalt. Cell culture supernatant can be passed through a column containing immobilized nickel ions, which binds the polyhistidine tag, whereas all untagged proteins pass through the column. The adapter module can be eluted with imidazole, which competes with the polyhistidine tag for binding to the column, or by a decrease in pH, which decreases the affinity of the tag for the resin. In an aspect, the adapter module is suitable for protein L based affinity chromatography (e.g. Capto L). In another aspect, adapter molecules may harbor a variable region of the heavy chain 3 family, which enables purification by column resins including a specific domain of staphylococcal protein A (e.g. MabSelect).

### Adapter module administration

One adapter module or more than one, like two, three, four or more adapter modules may be formulated for administration to a subject using techniques known to the skilled artisan.

Formulations containing one or more than one adapter module(s) may include pharmaceutically acceptable excipient(s). Excipients included in the formulations will have different purposes depending, for example, on the nature of the adapter modules and the mode of administration. Examples of generally used excipients include, without limitation: saline, buffered saline, dextrose, water-for- infection, glycerol, ethanol, and combinations thereof, stabilizing agents, solubilizing agents and surfactants, buffers and preservatives, tonicity agents, bulking agents, and lubricating agents. The formulations comprising adapter modules will typically have been prepared and cultured in the absence of any non-human components, such as animal serum (e.g., bovine serum albumin).

A formulation may include one adapter module or more than one, such as two, three, four, five, six or more adapter modules. adapter modules can vary based on the identity of the binding moiety, the identity of the tag, the mode of generation or a combination thereof. For example, a formulation may comprise adapter modules that recognize and bind to one, or more than one, such as two, three, four, five, six or more different human cell surface proteins, protein complexes or extracellular matrix structures.

Formulations comprising population(s) of RevCAR expressing immune cells may be incubated with a formulation including one or more adapter modules *ex vivo,* to decorate the RevCAR expressing immune cells with adapter modules before administration to a subject. Alternatively, formulations including one or more adapter modules can be administered directly to a subject or a combination of both strategies can be chosen. Route and dosage will vary between wide limits, depending upon the location, source, identity, extent and severity of the disease, the age and condition of the individual to be treated, etc. A physician will ultimately determine appropriate routes of application and dosages to be used.

Formulations comprising an adapter module are administered to a subject in an amount which is effective for treating and/or prophylaxis of the specific indication or disease. A typical dose-rate delivered per m² per day is between 1 ng to 1000 mg, preferably 5 ng to 1 mg, with dosages administered one or more times per day or week or continuously over a period of several weeks. However, the amount of adapter modules in formulations administered to a subject will vary between wide limits, depending upon the locatbn, source, identity, extent and severity of the cancer, the age and condition of the individual to be treated, etc. A physician will ultimately determine appropriate dosages to be used.

The present invention relates to methods of treating a subject having cancer, infections or autoimmune disorders, comprising administering to a subject in need of treatment one or more formulations of adapter module, wherein the adapter module binds to a cancer cell, and administering one or more therapeutically-effective populations of RevCAR expressing immune cells, wherein the RevCAR expressing immune cells bind the adapter module and induce cell death.

The term "cancer" is intended to be broadly interpreted and it encompasses all aspects of abnormal cell growth and/or cell division. Examples include: carcinoma, including but not limited to adenocarcinoma, squamous cell carcinoma, adenosquamous carcinoma, anaplastic carcinoma, large cell carcinoma, small cell carcinoma, and cancer of the skin, breast, prostate, bladder, vagina, cervix, uterus, liver, kidney, pancreas, spleen, lung, trachea, bronchi, colon, small intestine, stomach, esophagus, gall bladder; sarcoma, including but not limited to chondrosarcoma, Ewing's sarcoma, malignant hemangioendothelioma, malignant schwannoma, osteosarcoma, soft tissue sarcoma, and cancers of bone, cartilage, fat, muscle, vascular, and hematopoietic tissues; lymphoma and leukemia, including but not limited to mature B cell neoplasms, such as chronic lymphocytic leukemia/small lymphocytic lymphoma, B-cell prolymphocytic leukemia, lymphomas, and plasma cell neoplasms including multiple myeloma, mature T cell and natural killer (NK) cell neoplasms, such as T cell prolymphocytic leukemia, T cell large granular lymphocytic leukemia, aggressive NK cell leukemia, and adult T cell leukemia/lymphoma, Hodgkin lymphomas, and immunodeficiency-associated lymphoproliferative disorders; germ cell tumors, including but not limited to testicular and ovarian cancer; blastoma, including but not limited to hepatoblastoma, medulloblastoma, nephroblastoma, neuroblastoma, pancreatoblastoma, leuropulmonary blastoma and retinoblastoma. The term also encompasses benign tumors.

As used herein, the terms "treat", "treating", and "treatment" have their ordinary and customary meanings, and include one or more of: blocking, ameliorating, or decreasing in severity and/or frequency a symptom of cancer in a subject, and/or inhibiting the growth, division, spread, or proliferation of cancer cells, or progression of cancer (e.g., emergence of new tumors) in a subject. Treatment means blocking, ameliorating, decreasing, or inhibiting by about 1% to about 100% versus a subject in which the methods of the present invention have not been practiced. Preferably, the blocking, ameliorating, decreasing, or inhibiting is about 100%, 99%, 98%, 97%, 96%, 95%, 90%, 80%, 70%, 60%, 50%, 40%, 30%, 20%, 10%, 5% or 1% versus a subject in which the methods of the present invention have not been practiced.

Administration frequencies of both formulations comprising populations of RevCAR expressing immune cells and formulations of adapter modules will vary depending on factors that include the disease being treated, the elements comprising the RevCAR expressing immune cells and the adapter modules, and the modes of administration. Each formulation may be independently administered 4, 3, 2 or once daily, every other day, every third day, every fourth day, every fifth day, every sixth day, once weekly, every eight days, every nine days, every ten days, bi-weekly, monthly and bi-monthly.

The duration of treatment will be based on the disease being treated and will be best determined by the attending physician. However, continuation of treatment is contemplated to last for a number of days, weeks, or months.

The present invention offers flexibility in the methods of treatment, and as a result, the formulation(s) of adapter modules and the population(s) of RevCAR expressing immune cells may be administered to a subject in any order. Thus, the formulation(s) of adapter modules may be administered to a subject before, after or concurrently with the population(s) of RevCAR expressing immune cells. Alternatively, where more than one formulation of adapter modules and/or more than one population of RevCAR expressing immune cells are administered to a subject, the administration can be staggered. For example, a first formulation of adapter modules can be administered, followed by a first population of RevCAR expressing immune cells, which is then followed by a second formulation of tagged proteins and then a second population of RevCAR expressing immune cells.

The present invention also includes methods whereby a population of RevCAR expressing immune cells is coated with adapter modules prior to administration of the RevCAR expressing immune cells to the subject.

In each of the embodiments of the present invention the subject receiving treatment is a human or non-human animal, e.g., a non-human primate, bird, horse, cow, goat, sheep, a companion animal, such as a dog, cat or rodent, or other mammal.

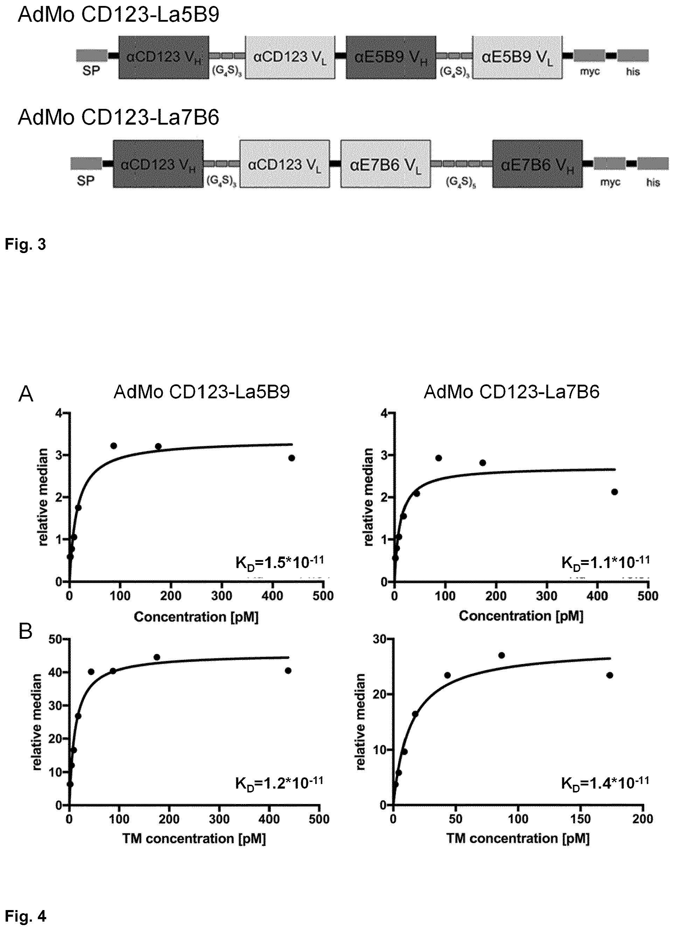

In an embodiment RevCAR genetically engineered T cells can be specifically redirected against tumor cells expressing CD123, a surface marker frequently detected in >80% of acute myeloid leukemia (AML) and nearly 100% of acute lymphoblastic leukemia (ALL). For this purpose, two CD123-specific adapter modules were designed as depicted in Fig. 3, the cDNA synthesized and cloned into a lentiviral vector and a stable CHO production cell line generated by lentiviral gene transfer. Adapter modules were purified from cell supernatant via the C-terminal His-tag using standard immobilized metal affinity chromatography (IMAC). One adapter module variant harbored an active scFv binding domain recognizing the La 5B9 epitope; alternatively the other adapter module comprised a scFv recognizing the La 7B6 epitope (Fig. 3). Both modules were binding to CD123-expressing AML cells with a comparable affinity of 15 pM and 11 pM, respectively (Fig. 4A). Binding towards the RevCAR tags could also be confirmed by binding experiments towards RevCAR expressing target cells (Fig. 4B). Affinity of both adapter moduls towards their specific RevCAR tag was comparable with a K_{D} of 12 pM for the La5B9 RevCAR tag and 14 pM for the La7B6 RevCAR tag (Fig. 4B).

For redirection of immune cells with the CD123-specific adaptor modules, two RevCARs were constructed harboring either the La 5B9 or the La 7B6 epitope as RevCAR tags. Human native T cells were genetically engineered to express either of the two RevCARs by means of lentiviral gene transfer. Upon incubation with CD123-expressing AML blast, primary human RevCAR T cells mediated specific lysis of AML blast in the presence of the corresponding adaptor molecule in a concentration depending fashion (Fig. 5).

In a further embodiment a nucleic acid encoding a reversed universal chimeric antigen receptor referred to as RevCAR1 according to SEQ. ID 1 is provided. The nucleic acid sequence encodes a human IL-2m leader peptide according to SEQ. ID 2, a human La 5B9 epitope according to SEQ. ID 3, a human CD28 portion according to SEQ. ID 4 to 6, including a human CD28 extracellular part with mutated binding motif according to SEQ. ID 4, a CD28 transmembrane domain according to SEQ. ID 5, and a human CD28 intracellular part including a mutated internalization motif according to SEQ. ID 6, and a human CD3 zeta intracellular domain according to SEQ. ID 7.

The product of the protein expression of the nucleic acid according to SEQ. ID 1 can be obtained in SEQ. ID 24.

The nucleic acid sequence of human La 5B9 epitope according to SEQ. ID 3 encodes for a protein domain according to SEQ. ID 28.

In a further embodiment a nucleic acid sequence encoding a reversed universal chimeric antigen receptor referred to as RevCAR2 according to SEQ. ID 8 is provided. The nucleic acid sequence encodes a human IL-2m leader peptide according to SEQ. ID 2, a human La 5B9 epitope according to SEQ. ID 3, an extracellular hinge and a transmembrane region of the human CD28 chain according to SEQ. ID 4 and 5, a human CD137 intracellular signaling domain according to SEQ. ID 9, and a human CD3 zeta intracellular domain according to SEQ. ID 7.

The product of the protein expression of the isolated nucleic acid sequence according to SEQ. ID 8 can be obtained in SEQ. ID 25.

In a further embodiment a nucleic acid sequence encoding a reversed universal chimeric antigen receptor referred to as RevCAR3 according to SEQ. ID 10 is provided. The nucleic acid sequence encodes a human IL-2m leader peptide according to SEQ. ID 2, a human La 7B6 epitope according to SEQ. ID 11, a human CD28 portion according to SEQ. ID 4 to 6, including a human CD28 extracellular part with mutated binding motif according to SEQ. ID 4, a CD28 transmembrane domain according to SEQ. ID 5, and a human CD28 intracellular part including a mutated internalization motif according to SEQ. ID 6, and a human CD3 zeta intracellular domain according to SEQ. ID 7.

The product of the protein expression of the isolated nucleic acid sequence according to SEQ. ID 10 can be obtained in SEQ. ID 26.

The nucleic acid sequence of human La 7B6 epitope according to SEQ. ID 11 encodes for a protein domain according to SEQ. ID 29.

In a further embodiment a nucleic acid sequence encoding a reversed universal chimeric antigen receptor referred to as RevCAR4 according to SEQ. ID 12 is provided. The nucleic acid sequence encodes a human IL-2m leader peptide according to SEQ. ID 2, a human La 7B6 epitope according to SEQ. ID 11, an extracellular hinge and a transmembrane region of the human CD28 chain according to SEQ. ID 4 and 5, a human CD137 intracellular signaling domain according to SEQ. ID 9, and a human CD3 zeta intracellular domain according to SEQ. ID 7.

The product of the protein expression of the isolated nucleic acid sequence according to SEQ. ID 12 can be obtained in SEQ. ID 27.

In a further embodiment of the invention an adapter module with a binding moiety for prostate specific membrane antigen PSMA is provided. The nucleic acid encoding this adapter module comprises sequences encoding an IgG kappa leader peptide according to SEQ. ID 13, a humanized heavy chain of an anti-La 5B9 scFv according to SEQ. ID 14, a humanized light chain of an anti-La 5B9 scFv according to SEQ. ID 15, a humanized heavy chain of an anti-PSMA scFv according to SEQ. ID 20, a humanized light chain of an anti-PSMA scFv according to SEQ. ID 21, a myc tag according to SEQ. ID 16 and a his tag according to SEQ. ID 17.

In a further embodiment of the invention an adapter module with a binding moiety for prostate specific membrane antigen PSMA is provided. The nucleic acid encoding this adapter module comprises sequences encoding an IgG kappa leader peptide according to SEQ. ID 13, a humanized heavy chain of an anti-La 7B6 scFv according to SEQ. ID 18, a humanized light chain of an anti-La 7B6 scFv according to SEQ. ID 19, a humanized heavy chain of an anti-PSMA scFv according to SEQ. ID 20, a humanized light chain of an anti-PSMA scFv according to SEQ. ID 21, a myc tag according to SEQ. ID 16 and a his tag according to SEQ. ID 17.

In a further embodiment of the invention an adapter module with a binding moiety for leukemia antigen CD123 is provided. The nucleic acid encoding this adapter module comprises sequences encoding an IgG kappa leader peptide according to SEQ. ID 13, a humanized heavy chain of an anti-La 5B9 scFv according to SEQ. ID 14, a humanized light chain of an anti-La 5B9 scFv according to SEQ. ID 15, a humanized heavy chain of an anti-CD123 scFv according to SEQ. ID 22, a humanized light chain of an anti-CD123 scFv according to SEQ. ID 23, a myc tag according to SEQ. ID 16 and a his tag according to SEQ. ID 17.

In a further embodiment of the invention an adapter module with a binding moiety for leukemia antigen CD123 is provided. The nucleic acid encoding this adapter module comprises sequences encoding an IgG kappa leader peptide according to SEQ. ID 13, a humanized heavy chain of an anti-La 7B6 scFv according to SEQ. ID 18, a humanized light chain of an anti-La 7B6 scFv according to SEQ. ID 19, a humanized heavy chain of an anti-CD123 scFv according to SEQ. ID 22, a humanized light chain of an anti-CD123 scFv according to SEQ. ID 23, a myc tag according to SEQ. ID 16 and a his tag according to SEQ. ID 17.

The nucleic acid sequence of humanized anti-La 5B9 variable region heavy chain according to SEQ. ID 14 encodes for a protein according to SEQ. ID 30, whereas the humanized anti-La 5B9 variable region light chain according to SEQ. ID 15 encodes for a protein according to SEQ. ID 31.

The nucleic acid sequence of the myc tag according to SEQ. ID 16 encodes for a protein according to SEQ. ID 32, whereas the his tag according to SEQ. ID 17 encodes for a protein according to SEQ. ID 33.

The nucleic acid sequence of humanized anti-7B6 variable region heavy chain according to SEQ. ID 18 encodes for a protein according to SEQ. ID 34, whereas the humanized anti-7B6 variable region light chain according to SEQ. ID 19 encodes for a protein according to SEQ. ID 35.

The nucleic acid sequence of humanized anti-PSMA variable region heavy chain according to SEQ. ID 20 encodes for a protein according to SEQ. ID 36, whereas the humanized anti- PSMA variable region light chain according to SEQ. ID 21 encodes for a protein according to SEQ. ID 37.

The nucleic acid sequence of humanized anti-CD123 variable region heavy chain according to SEQ. ID 22 encodes for a protein according to SEQ. ID 38, whereas the humanized anti-CD123 variable region light chain according to SEQ. ID 23 encodes for a protein according to SEQ. ID 39.

## Claims

1. A nucleic acid encoding a reversed universal chimeric antigen receptor, wherein the receptor comprises three domains, wherein
- the first domain is a peptide epitope tag,
- the second domain is an extracellular hinge and a transmembrane domain and
- the third domain is a signal transduction domain,
wherein the peptide epitope tag binds to a tag-binding domain.

2. The nucleic acid according to claim 1, wherein the peptide epitope tag is a short linear epitope from a human protein.

3. The nucleic acid according to claim 1 and 2, wherein the peptide epitope tag is a short linear epitope from a human nuclear protein.

4. The nucleic acid according to claim 1 to 3, wherein the peptide epitope tag is a short linear epitope from the human nuclear La protein including but not restricted to E5B9 or E7B6 according to SEQ. ID 3 and 11.

5. The nucleic acid according to any of claims 1 to 4, wherein the hinge and transmembrane region is selected from hinge and transmembrane regions of human CD28 molecule, CD8a chain, NK cell receptors, preferably natural killer group NKG2D, DAP12, or parts of the constant region of an antibody as well as combinations of different hinge and transmembrane domains thereof.

6. The nucleic acid according to any of claims 1 to 5, wherein the signal transduction domain is selected from cytoplasmic regions of CD28, CD137 (41BB), CD134 (OX40), DAP10 and CD27, programmed cell death-1 (PD-1), cytotoxic T-lymphocyte antigen 4 (CTLA-4) and cytoplasmic regions of CD3 chains, DAP12 and T cell activation inducing Fc receptors.

7. The nucleic acid according to any of claims 1 to 6, wherein the receptor comprises a fourth domain, which is a short peptide linker in the extracellular portion of the receptor.

8. The nucleic acid according to any of claims 1 to 6 according to SEQ. ID 1, 8, 10 and 12.

9. An adapter module composed of a binding moiety specific for a certain human cell surface protein or protein complex and a tag-binding domain, which is directed against the peptide epitope tag of the reversed universal chimeric antigen receptor.

10. The adapter module according to claim 9, wherein the binding moiety of the adapter module includes an antibody or fragment thereof that binds to surface antigens CD2, CD3, CD4, CD8, CD10, CD19, CD20, CD22, CD23, CD25, CD30, CD33, CD38, CD44, CD52, CD90, CD99, CD123, CD181, CD182, CD184, CD223, CD269, CD274, CD276, CD279 and CD366, interleukin receptors, especially preferred IL-8Rα (CXCR1), IL-8Rβ (CXCR2), IL-11Rα, IL-11Rβ, IL-13Rα1 and 2, CXCR4, members of the epidermal growth factor receptor family, especially preferred ErbB1, ErbB2, ErbB3, ErbB4 and mutants thereof, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the mucin protein family, follicle stimulating hormone receptor (FSHR) the human high molecular weight-melanoma-associated antigen (HMW-MAA), folate binding protein FBP, a-Folate receptor, ligands of the NKG2D receptor, cytokine receptors, members of the epithelia glycoprotein family, diasialogangliosides, members of the carbonic anhydrase family, members of the carbohydrate antigen family, Notch ligands, melanoma-associated chondroitin sulfate proteoglycan (MCSP), glycoprotein A33, and tumor-specific glycans, including mutants of the named proteins and protein families. In addition, the binding moiety of adapter modules include, but are not limited to, antibodies or fragments thereof that binds to cytoplasmic or nuclear antigens like the La/SSB antigen, members of the Rho family of GTPases, members of the high mobility group proteins and others.

11. The adapter module according to claim 9, wherein the binding moiety of the adapter module is composed of the alpha and beta or the gamma and delta chains of a T cell receptor (TCR) or fragments thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens and leukemia-associated antigens.

12. The adapter module according to claim 9, wherein the binding moiety of the adapter module comprises a ligand to a protein or protein complex, further on referred as receptor, or a fragment thereof. Such ligands may bind to, but are not limited to, cytokine receptors, ligands of the NKG2D receptor, ligands to the EGFR family members, ligands of checkpoints molecules like PD-1, CTLA-4, lymphocyte-activation gene 3 (LAG-3) or T-cell immunoglobulin and mucin-domain containing-3 (TIM-3), or auto-reactive TCRs.

13. The adapter module according to claim 9, wherein the binding moiety of the adapter module comprises a chemically synthesized peptide derivative fused to the tag binding moiety via a chemical reaction (i.e. click chemistry). Such ligands may have binding specificity to, but are not limited to, CD2, CD3, CD4, CD8, CD10, CD19, CD20, CD22, CD23, CD25, CD30, CD33, CD38, CD44, CD52, CD90, CD99, CD123, CD181, CD182, CD184, CD223, CD269, CD274, CD276, CD279 and CD366, interleukin receptors, especially preferred IL-8Rα (CXCR1), IL-8Rβ (CXCR2), IL-11Rα, IL-11Rβ, IL-13Rα1 and 2, CXCR4; c-Met, transforming growth factor β receptors, members of the epidermal growth factor receptor family, especially preferred ErbB1, ErbB2, ErbB3, ErbB4 and mutants thereof, ephrin receptors, especially preferred EphA1-10, EphA5 or EphB1-6; prostate stem cell antigen (PSCA), prostate specific membrane antigen (PSMA), carcinoembryonic antigen (CEA), fetal acetylcholine receptor, onco-fetal antigens, tumor-specific glycans, especially preferred Tn or sialyl-Tn (sTn); VEGFR 1, VEGFR 2 or VEGFR 3, Neuropilin-1, epithelia cell adhesion molecule (EpCAM), epidermal growth factor receptor (EGFR), alphafetoprotein (AFP), mucins, especially preferred MUC1, MUC16 or MUC18; follicle stimulating hormone receptor (FSHR), human high molecular weight-melanoma-associated antigen (HMW-MAA), folate binding protein (FBP), a folate receptor, NKG2D, major histocompatibility complex (MHC) class I molecules, especially preferred MHC class I chain-related gene A (MICA) or B (MICB), UL16 binding protein (ULPB) 1, ULPB 2, ULPB 3, ribonucleic acid export 1 (Rae-1) family members or histocompatibility 60 (H-60); chaperones and heat shock proteins, especially preferred heat shock protein (HSP) 90 or 78 kDa glucose-regulated protein (GRP78); EGP-2 or EGP-4, diasialoganglioside 2 (GD2) or GD3, carbonic anhydrase 9 (CAIX), Lewis Y (LeY), C-type lectin-like molecule-1 (CLL-1), tumor necrosis factor related apoptosis inducing ligand (TRAIL) receptor, apoptosis antigen 1 (APO-1, Fas, CD95), members of the keratin family or integrins, especially preferred avβ3 or avβ5, aminopeptidase A, aminopeptidase N or neural/glial antigen 2 (NG2), Notch ligands.

14. The adapter module according to any of claims 9 to 13, wherein the binding moiety of adapter modules comprise bi- and multispecific antigen specificities including binding to PSCA and PSMA antigen, CD19 and CD20 antigen, CD19, CD20, and CD22 antigen, CD33 and CD123 antigen, CD33 and CD99, CD33 and TIM-3, ErbB-1 and -2, PSCA and ErbB-2.

15. A nucleic acid encoding an adapter module according to any of claims 9 to 14, wherein the tag binding moieties of adapter modules include antibodies or fragments thereof that bind to the 5B9 or 7B6 epitopes of the La/SSB antigen, preferably according to SEQ. ID 14, 15, 18 and 19.

16. A nucleic acid encoding an adapter module according to any of claims 9 to 14, wherein the target binding moieties of adapter modules include antibodies or fragments thereof that bind to PSMA or CD123, preferably according to SEQ. ID 20, 21, 22 and 23.

17. A cell or vector comprising a nucleic acid according to any of claims 1 to 8.

18. The cell according to claim 17, wherein the cell is selected from the group of immune cells including T cell, a Natural Killer cell, a cytotoxic T lymphocyte, and a regulatory T cell.

19. A kit comprising:
- a vector according to claim 17 and
- an adapter module according to any of claims 9 to 14 and/or a vector encoding an nucleic acid according to claims 15 and 16.

20. A Formulation comprising RevCAR expressing immune cells according to claim 17 or 18 and/or an adapter module according to any of claims 9 to 14 for administration to a subject.

21. Use of a cell according to any of claims 17 or 18 and an adapter module according to any of claims 9 to 14 for stimulating a reversed universal chimeric antigen receptor mediated immune response in a mammal.
